# EUROPEAN PATENT APPLICATION

(11) **EP 1 656 951 A1**
(43) Date of publication of application: **17.05.2006**
(21) Application number: 04026934.2
(22) Date of filing: 12.11.2004
(51) Int. Cl.: A61K 47/48, C07K 7/06, A61P 31/18, A61P 35/00

(54) **Conjugates with enhanced cell uptake activity**

(71) Applicant: Xigen S.A., 1005 Lausanne (CH)
(72) Inventor: Bonny, Christophe, ch-1006 Lausanne (CH); Coquoz, Didier, CH-1025 St Sulpice (CH); Chen, Jianhua, CH-1010 Lausanne (CH)
(74) Representative: Graf von Stosch, Andreas

(57) **Abstract**

This invention relates to a conjugate molecule comprising at least one first portion (I) comprising a carrier sequence and at least one second portion (II) comprising at lest one anti-tumot drug molecule or a protease inhibitor molecule, said conjugate molecule comprising D-enantiomeric amino acids in its portion (I). Furthermore, the invention relates to pharmaceutical compositions containing said conjugate molecule as well as to the use of said conjugate molecule for therapeutical treatment. Methods for improving cell permeability are disclosed as well.

SEQUENCE LISTING

<110> XIGEN S.A.

<120> Conjugate Molecule Compounds with Enhanced Cell Uptake Activity

<130> CX01P003EP

<140> EP 04 026 934.2
<141> 2004-11-12

<160> 45

<170> PatentIn version 3.3

<210> 1
<211> 1
<212> PRT
<213> Artificial

<220>
<223> portion (I) ("carrier" or "trafficking" sequence)


<220>
<221> misc_feature
<223> Description of sequence: general formula NH2-Xm-COOH, whereby m
      is an integer between 3 and 40, preferably 3 and 20 and most
      preferably between 3 and 12; (see page 3);

<220>
<221> REPEAT
<222> (1)..(1)
<223> 3 to 40, preferably 3 to 20, most preferably 3 to 12

<220>
<221> MISC_FEATURE
<222> (1)..(1)
<223> x is selected from D-amino acids arginine or lysine

<400> 1

Xaa
1


<210> 2
<211> 3
<212> PRT
<213> Artificial

<220>
<223> portion (I) ("carrier" or "trafficking" sequence)


<220>
<221> misc_feature
<223> Description of sequence: general formula NH2-XnArXn-COOH, "n"
      and "r" represent an integer from 1 to 20, preferably 3 to 10 and
      more preferably from 3 to 6; (see page 4);

<220>
<221> misc_feature

<222>  (1)..(1)
<223>  Xaa is selected from D-amino acids arginine or lysine

<220>
<221> .REPEAT
<222>  (1)..(1)
<223>  1 to 20,  preferably 3 to 10, more preferably 3 to 6

<220>
<221>  misc_feature
<222>  (2)..(2)
<223>  A is selected from any non-basic D-amino acid

<220>
<221>  REPEAT
<222>  (2)..(2)
<223>  1 to 20,  preferably 3 to 10, more preferably 3 to 6

<220>
<221>  misc_feature
<222>  (3)..(3)
<223>  Xaa is selected from D-amino acids arginine or lysine

<220>
<221>  REPEAT
<222>  (3)..(3)
<223>  1 to 20,  preferably 3 to 10, more preferably 3 to 6

<400>  2

Xaa Xaa Xaa
1


<210>  3
<211>  5
<212>  PRT
<213>  Artificial

<220>
<223>  portion (I) ("carrier" or "trafficking" sequence)


<220>
<221>  misc_feature
<223>  Description of sequence:  general formula:  NH2-XpAoXpAoXp-COOH,
       wherein "o" is an integer from 0 to 14 and "p" is an integer,
       independent from "o", from 0 to 14; (see page 4);

<220>
<221>  REPEAT
<222>  (1)..(1)
<223>  0 to 14

<220>
<221>  misc_feature
<222>  (1)..(1)
<223>  D-amino acids arginine or lysine

<220>
<221>  REPEAT
<222>  (2)..(2)
<223>  0 to 14

<220>
<221> misc_feature
<222> (2)..(2)
<223> amino acid is selected from any non-basic D-amino acid

<220>
<221> REPEAT
<222> (3)..(3)
<223> 0 to 14

<220>
<221> misc_feature
<222> (3)..(3)
<223> D-amino acids arginine or lysine

<220>
<221> REPEAT
<222> (4)..(4)
<223> 0 to 14

<220>
<221> misc_feature
<222> (4)..(4)
<223> amino acid is selected from any non-basic D-amino acid

<220>
<221> REPEAT
<222> (5)..(5)
<223> 0 to 14

<220>
<221> misc_feature
<222> (5)..(5)
<223> amino acid is selected from D-amino acids arginine or lysine

<400> 3

Xaa Xaa Xaa Xaa Xaa
1               5


<210> 4
<211> 5
<212> PRT
<213> Artificial

<220>
<223> portion (I) ("carrier" or "trafficking" sequence)


<220>
<221> misc_feature
<223> Description of sequence: general formula: NH2-AoXpAoXpAo-COOH,
      wherein "o" is an integer from 0 to 14 and "p" is an integer,
      independent from "o", from 0 to 14; (see page 4);

<220>
<221> misc_feature
<222> (1)..(1)
<223> amino acid is selected from any non-basic D-amino acid

<220>

<221>  REPEAT
<222>  (1)..(1)
<223>  0 to 14

<220>
<221>  misc_feature
<222>  (2)..(2)
<223>  amino acid is selected from D-amino acids arginine or lysine

<220>
<221>  REPEAT
<222>  (2)..(2)
<223>  0 to 14

<220>
<221>  misc_feature
<222>  (3)..(3)
<223>  amino acid is selected from any non-basic D-amino acid

<220>
<221>  REPEAT
<222>  (3)..(3)
<223>  0 to 14

<220>
<221>  misc_feature
<222>  (4)..(4)
<223>  amino acid is selected from D-amino acids arginine or lysine

<220>
<221>  REPEAT
<222>  (4)..(4)
<223>  0 to 14

<220>
<221>  misc_feature
<222>  (5)..(5)
<223>  amino acid is selected from any non-basic D-amino acid

<220>
<221>  REPEAT
<222>  (5)..(5)
<223>  0 to 14

<400>  4

Xaa Xaa Xaa Xaa Xaa
1               5


<210>  5
<211>  6
<212>  PRT
<213>  Artificial

<220>
<223>  portion (I) ("carrier" or "trafficking" sequence)


<220>
<221>  misc_feature
<223>  Description of sequence:  general formula:
       NH2-XpAoXpAoXpAo-COOH, wherein "o" is an integer from 0 to 14 and

"p" is an integer, independent from "o", from 0 to 14; (see page
4);

<220>
<221> REPEAT
<222> (1)..(1)
<223> 0 to 14

<220>
<221> misc_feature
<222> (1)..(1)
<223> amino acid is selected from D-amino acids arginine or lysine

<220>
<221> REPEAT
<222> (2)..(2)
<223> 0 to 14

<220>
<221> misc_feature
<222> (2)..(2)
<223> amino acid is selected from any non-basic D-amino acid

<220>
<221> REPEAT
<222> (3)..(3)
<223> 0 to 14

<220>
<221> misc_feature
<222> (3)..(3)
<223> amino acid is selected from D-amino acids arginine or lysine

<220>
<221> REPEAT
<222> (4)..(4)
<223> 0 to 14

<220>
<221> misc_feature
<222> (4)..(4)
<223> amino acid is selected from any non-basic D-amino acid

<220>
<221> REPEAT
<222> (5)..(5)
<223> 0 to 14

<220>
<221> misc_feature
<222> (5)..(5)
<223> amino acid is selected from D-amino acids arginine or lysine

<220>
<221> REPEAT
<222> (6)..(6)
<223> 0 to 14

<220>
<221> misc_feature
<222> (6)..(6)
<223> amino acid is selected from any non-basic D-amino acid

<400>  5

Xaa Xaa Xaa Xaa Xaa Xaa
1               5


<210>  6
<211>  9
<212>  PRT
<213>  Artificial

<220>
<223>  portion (I) ("carrier" or "trafficking" sequence)


<220>
<221>  misc_feature
<223>  Description of sequence:  general formula:
       NH2-AoXpAoXpAoXpAoXpAo-COOH, wherein "o" is an integer from 0 to
       14 and "p" is an integer, independent from "o", from 0 to 14;
       (see page 4);

<220>
<221>  REPEAT
<222>  (1)..(1)
<223>  0 to 14

<220>
<221>  misc_feature
<222>  (1)..(1)
<223>  amino acid is selected from any non-basic D-amino acid

<220>
<221>  REPEAT
<222>  (2)..(2)
<223>  0 to 14

<220>
<221>  misc_feature
<222>  (2)..(2)
<223>  amino acid is selected from D-amino acids arginine or lysine

<220>
<221>  REPEAT
<222>  (3)..(3)
<223>  0 to 14

<220>
<221>  misc_feature
<222>  (3)..(3)
<223>  amino acid is selected from any non-basic D-amino acid

<220>
<221>  REPEAT
<222>  (4)..(4)
<223>  0 to 14

<220>
<221>  misc_feature
<222>  (4)..(4)
<223>  amino acid is selected from D-amino acids arginine or lysine

<220>

<221>  REPEAT
<222>  (5)..(5)
<223>  0 to 14

<220>
<221>  misc_feature
<222>  (5)..(5)
<223>  amino acid is selected from any non-basic D-amino acid

<220>
<221>  REPEAT
<222>  (6)..(6)
<223>  0 to 14

<220>
<221>  misc_feature
<222>  (6)..(6)
<223>  amino acid is selected from D-amino acids arginine or lysine

<220>
<221>  REPEAT
<222>  (7)..(7)
<223>  0 to 14

<220>
<221>  misc_feature
<222>  (7)..(7)
<223>  amino acid is selected from any non-basic D-amino acid

<220>
<221>  REPEAT
<222>  (8)..(8)
<223>  0 to 14

<220>
<221>  misc_feature
<222>  (8)..(8)
<223>  amino acid is selected from D-amino acids arginine or lysine

<220>
<221>  REPEAT
<222>  (9)..(9)
<223>  0 to 14

<220>
<221>  misc_feature
<222>  (9)..(9)
<223>  amino acid is selected from any non-basic D-amino acid

<400>  6

Xaa Xaa Xaa Xaa Xaa Xaa Xaa Xaa Xaa
1               5


<210>  7
<211>  11
<212>  PRT
<213>  Artificial

<220>
<223>  portion (I) ("carrier" or "trafficking" sequence)

<220>
<221> misc_feature
<223> Description of sequence: general formula:
NH2-AoXpAoXpAoXpAoXpAoXpAo-COOH, wherein "o" is an integer from 0
to 14 and "p" is an integer, independent from "o", from 0 to 14;
(see page 4);

<220>
<221> REPEAT
<222> (1)..(1)
<223> 0 to 14

<220>
<221> misc_feature
<222> (1)..(1)
<223> any non-basic D-amino acid

<220>
<221> REPEAT
<222> (2)..(2)
<223> 0 to 14

<220>
<221> misc_feature
<222> (2)..(2)
<223> D-amino acids arginine or lysine

<220>
<221> REPEAT
<222> (3)..(3)
<223> 0 to 14

<220>
<221> misc_feature
<222> (3)..(3)
<223> any non-basic D-amino acid

<220>
<221> REPEAT
<222> (4)..(4)
<223> 0 to 14

<220>
<221> misc_feature
<222> (4)..(4)
<223> D-amino acids arginine or lysine

<220>
<221> REPEAT
<222> (5)..(5)
<223> 0 to 14

<220>
<221> misc_feature
<222> (5)..(5)
<223> any non-basic D-amino acid

<220>
<221> REPEAT
<222> (6)..(6)
<223> 0 to 14

<220>
<221> misc_feature
<222> (6)..(6)
<223> D-amino acids arginine or lysine

<220>
<221> REPEAT
<222> (7)..(7)
<223> 0 to 14

<220>
<221> misc_feature
<222> (7)..(7)
<223> any non-basic D-amino acid

<220>
<221> REPEAT
<222> (8)..(8)
<223> 0 to 14

<220>
<221> misc_feature
<222> (8)..(8)
<223> D-amino acids arginine or lysine

<220>
<221> REPEAT
<222> (9)..(9)
<223> 0 to 14

<220>
<221> misc_feature
<222> (9)..(9)
<223> any non-basic D-amino acid

<220>
<221> REPEAT
<222> (10)..(10)
<223> 0 to 14

<220>
<221> misc_feature
<222> (10)..(10)
<223> D-amino acids arginine or lysine

<220>
<221> REPEAT
<222> (11)..(11)
<223> 0 to 14

<220>
<221> misc_feature
<222> (11)..(11)
<223> any non-basic D-amino acid

<400> 7

Xaa Xaa Xaa Xaa Xaa Xaa Xaa Xaa Xaa Xaa Xaa
1               5                   10


<210> 8
<211> 13

<212>  PRT
<213>  Artificial

<220>
<223>  portion (I) ("carrier" or "trafficking" sequence)


<220>
<221>  misc_feature
<223>  Description of sequence:  general formula:
       NH2-AoXpAoXpAoXpAoXpAoXpAoXpAo-COOH, wherein "o" is an integer
       from 0 to 14 and "p" is an integer, independent from "o", from 0
       to 14; (see page 4);

<220>
<221>  REPEAT
<222>  (1)..(1)
<223>  0 to 14

<220>
<221>  misc_feature
<222>  (1)..(1)
<223>  amino acid is selected from any non-basic D-amino acid

<220>
<221>  REPEAT
<222>  (2)..(2)
<223>  0 to 14

<220>
<221>  misc_feature
<222>  (2)..(2)
<223>  amino acid is selected from D-amino acids arginine or lysine

<220>
<221>  REPEAT
<222>  (3)..(3)
<223>  0 to 14

<220>
<221>  misc_feature
<222>  (3)..(3)
<223>  amino acid is selected from any non-basic D-amino acid

<220>
<221>  REPEAT
<222>  (4)..(4)
<223>  0 to 14

<220>
<221>  misc_feature
<222>  (4)..(4)
<223>  amino acid is selected from D-amino acids arginine or lysine

<220>
<221>  REPEAT
<222>  (5)..(5)
<223>  0 to 14

<220>
<221>  misc_feature
<222>  (5)..(5)
<223>  amino acid is selected from any non-basic D-amino acid

<220>
<221> REPEAT
<222> (6)..(6)
<223> 0 to 14

<220>
<221> misc_feature
<222> (6)..(6)
<223> amino acid is selected from D-amino acids arginine or lysine

<220>
<221> REPEAT
<222> (7)..(7)
<223> 0 to 14

<220>
<221> misc_feature
<222> (7)..(7)
<223> amino acid is selected from any non-basic D-amino acid

<220>
<221> REPEAT
<222> (8)..(8)
<223> 0 to 14

<220>
<221> misc_feature
<222> (8)..(8)
<223> amino acid is selected from D-amino acids arginine or lysine

<220>
<221> REPEAT
<222> (9)..(9)
<223> 0 to 14

<220>
<221> misc_feature
<222> (9)..(9)
<223> amino acid is selected from any non-basic D-amino acid

<220>
<221> REPEAT
<222> (10)..(10)
<223> 0 to 14

<220>
<221> misc_feature
<222> (10)..(10)
<223> amino acid is selected from D-amino acids arginine or lysine

<220>
<221> REPEAT
<222> (11)..(11)
<223> 0 to 14

<220>
<221> misc_feature
<222> (11)..(11)
<223> amino acid is selected from any non-basic D-amino acid

<220>
<221> REPEAT

<222>  (12)..(12)
<223>  0 to 14

<220>
<221>  REPEAT
<222>  (13)..(13)
<223>  0 to 14

<220>
<221>  misc_feature
<222>  (13)..(13)
<223>  amino acid is selected from any non-basic D-amino acid

<400>  8

Xaa Xaa Xaa Xaa Xaa Xaa Xaa Xaa Xaa Xaa Xaa Xaa Xaa
1               5                   10


<210>  9
<211>  15
<212>  PRT
<213>  Artificial

<220>
<223>  portion (I) ("carrier" or "trafficking" sequence)


<220>
<221>  misc_feature
<223>  Description of sequence:  general formula:
       NH2-AoXpAoXpAoXpAoXpAoXpAoXpAoXpAo-COOH, wherein "o" is an
       integer from 0 to 14 and "p" is an integer, independent from "o",
       from 0 to 14; (see page 4);

<220>
<221>  REPEAT
<222>  (1)..(1)
<223>  0 to 14

<220>
<221>  misc_feature
<222>  (1)..(1)
<223>  amino acid is selected from any non-basic D-amino acid

<220>
<221>  REPEAT
<222>  (2)..(2)
<223>  0 to 14

<220>
<221>  misc_feature
<222>  (2)..(2)
<223>  amino acid is selected from D-amino acids arginine or lysine

<220>
<221>  REPEAT
<222>  (3)..(3)
<223>  0 to 14

<220>
<221>  misc_feature
<222>  (3)..(3)

<223>  amino acid is selected from any non-basic D-amino acid

<220>
<221>  REPEAT
<222>  (4)..(4)
<223>  0 to 14

<220>
<221>  misc_feature
<222>  (4)..(4)
<223>  amino acid is selected from D-amino acids arginine or lysine

<220>
<221>  REPEAT
<222>  (5)..(5)
<223>  0 to 14

<220>
<221>  misc_feature
<222>  (5)..(5)
<223>  amino acid is selected from any non-basic D-amino acid

<220>
<221>  REPEAT
<222>  (6)..(6)
<223>  0 to 14

<220>
<221>  misc_feature
<222>  (6)..(6)
<223>  amino acid is selected from D-amino acids arginine or lysine

<220>
<221>  REPEAT
<222>  (7)..(7)
<223>  0 to 14

<220>
<221>  misc_feature
<222>  (7)..(7)
<223>  amino acid is selected from any non-basic D-amino acid

<220>
<221>  REPEAT
<222>  (8)..(8)
<223>  0 to 14

<220>
<221>  misc_feature
<222>  (8)..(8)
<223>  amino acid is selected from D-amino acids arginine or lysine

<220>
<221>  REPEAT
<222>  (9)..(9)
<223>  0 to 14

<220>
<221>  misc_feature
<222>  (9)..(9)
<223>  amino acid is selected from any non-basic D-amino acid

<220>

<221>  REPEAT
<222>  (10)..(10)
<223>  0 to 14

<220>
<221>  misc_feature
<222>  (10)..(10)
<223>  amino acid is selected from D-amino acids arginine or lysine

<220>
<221>  REPEAT
<222>  (11)..(11)
<223>  0 to 14

<220>
<221>  misc_feature
<222>  (11)..(11)
<223>  amino acid is selected from any non-basic D-amino acid

<220>
<221>  REPEAT
<222>  (12)..(12)
<223>  0 to 14

<220>
<221>  misc_feature
<222>  (12)..(12)
<223>  amino acid is selected from D-amino acids arginine or lysine

<220>
<221>  REPEAT
<222>  (13)..(13)
<223>  0 to 14

<220>
<221>  misc_feature
<222>  (13)..(13)
<223>  amino acid is selected from any non-basic D-amino acid

<220>
<221>  REPEAT
<222>  (14)..(14)
<223>  0 to 14

<220>
<221>  misc_feature
<222>  (14)..(14)
<223>  amino acid is selected from D-amino acids arginine or lysine

<220>
<221>  REPEAT
<222>  (15)..(15)
<223>  0 to 14

<220>
<221>  misc_feature
<222>  (15)..(15)
<223>  amino acid is selected from any non-basic D-amino acid

<400>  9

Xaa Xaa Xaa Xaa Xaa Xaa Xaa Xaa Xaa Xaa Xaa Xaa Xaa Xaa Xaa
1               5                   10                  15

<210>  10
<211>  4
<212>  PRT
<213>  Artificial

<220>
<223>  portion (I) ("carrier" or "trafficking" sequence)


<220>
<221>  misc_feature
<223>  Description of Sequence: NH2-KTRR-COOH; (see page 4);

<220>
<221>  misc_feature
<222>  (1)..(4)
<223>  all amino acids are D-enantiomeric amino acids

<400>  10

Lys Thr Arg Arg
1


<210>  11
<211>  4
<212>  PRT
<213>  Artificial

<220>
<223>  portion (I) ("carrier" or "trafficking" sequence)


<220>
<221>  misc_feature
<223>  Description of Sequence: NH2-RLKR-COOH; (see page 4);

<220>
<221>  misc_feature
<222>  (1)..(4)
<223>  all amino acids are D-enantiomeric amino acids

<400>  11

Arg Leu Lys Arg
1


<210>  12
<211>  4
<212>  PRT
<213>  Artificial

<220>
<223>  portion (I) ("carrier" or "trafficking" sequence)


<220>
<221>  misc_feature
<223>  Description of Sequence: NH2-KPRR-COOH; (see page 4);

<220>
<221>  misc_feature
<222>  (1)..(4)
<223>  all amino acids are D-enantiomeric amino acids

<400>  12

Lys Pro Arg Arg
1


<210>  13
<211>  5
<212>  PRT
<213>  Artificial

<220>
<223>  portion (I) ("carrier" or "trafficking" sequence)


<220>
<221>  misc_feature
<223>  Description of Sequence: NH2-KRFQR-COOH; (see page 4);

<220>
<221>  misc_feature
<222>  (1)..(5)
<223>  all amino acids are D-enantiomeric amino acids

<400>  13

Lys Arg Phe Gln Arg
1               5


<210>  14
<211>  5
<212>  PRT
<213>  Artificial

<220>
<223>  portion (I) ("carrier" or "trafficking" sequence)


<220>
<221>  misc_feature
<223>  Description of Sequence: NH2-GRIRR-COOH; (see page 4);

<220>
<221>  misc_feature
<222>  (1)..(5)
<223>  all amino acids are D-enantiomeric amino acids

<400>  14

Gly Arg Ile Arg Arg
1               5


<210>  15
<211>  7
<212>  PRT
<213>  Artificial

<220>
<223>  portion (I) ("carrier" or "trafficking" sequence)

<220>
<221>  misc_feature
<223>  Description of Sequence: NH2-NIGRRRN-COOH; (see page 4);

<220>
<221>  misc_feature
<222>  (1)..(7)
<223>  all amino acids are D-enantiomeric amino acids

<400>  15

Asn Ile Gly Arg Arg Arg Asn
1               5

<210>  16
<211>  7
<212>  PRT
<213>  Artificial

<220>
<223>  portion (I) ("carrier" or "trafficking" sequence)

<220>
<221>  misc_feature
<223>  Description of Sequence: NH2-RAGRNGR-COOH; (see page 4);

<220>
<221>  misc_feature
<222>  (1)..(7)
<223>  all amino acids are D-enantiomeric amino acids

<400>  16

Arg Ala Gly Arg Asn Gly Arg
1               5

<210>  17
<211>  4
<212>  PRT
<213>  Artificial

<220>
<223>  portion (I) ("carrier" or "trafficking" sequence)

<220>
<221>  misc_feature
<223>  Description of Sequence: NH2-RPRR-COOH; (see page 4);

<220>
<221>  misc_feature
<222>  (1)..(4)
<223>  all amino acids are D-enantiomeric amino acids

<400>  17

Arg Pro Arg Arg
1


<210>  18
<211>  5
<212>  PRT
<213>  Artificial

<220>
<223>  portion (I) ("carrier" or "trafficking" sequence)


<220>
<221>  misc_feature
<223>  Description of Sequence: NH2-GKRRG-COOH; (see page 4);

<220>
<221>  misc_feature
<222>  (1)..(5)
<223>  all amino acids are D-enantiomeric amino acids

<400>  18

Gly Lys Arg Arg Gly
1               5


<210>  19
<211>  4
<212>  PRT
<213>  Artificial

<220>
<223>  portion (I) ("carrier" or "trafficking" sequence)


<220>
<221>  misc_feature
<223>  Description of Sequence: NH2-KRRE-COOH; (see page 4);

<220>
<221>  misc_feature
<222>  (1)..(4)
<223>  all amino acids are D-enantiomeric amino acids

<400>  19

Lys Arg Arg Glu
1


<210>  20
<211>  7
<212>  PRT
<213>  Artificial

<220>
<223>  portion (I) ("carrier" or "trafficking" sequence)


<220>

<221>  misc_feature
<223>  Description of Sequence: NH2-RQKRGGS-COOH; (see page 4);

<220>
<221>  misc_feature
<222>  (1)..(7)
<223>  all amino acids are D-enantiomeric amino acids

<400>  20

Arg Gln Lys Arg Gly Gly Ser
1               5


<210>  21
<211>  4
<212>  PRT
<213>  Artificial

<220>
<223>  portion (I) ("carrier" or "trafficking" sequence)


<220>
<221>  misc_feature
<223>  Description of Sequence: NH2-RKSR-COOH; (see page 4);

<220>
<221>  misc_feature
<222>  (1)..(4)
<223>  all amino acids are D-enantiomeric amino acids

<400>  21

Arg Lys Ser Arg
1


<210>  22
<211>  5
<212>  PRT
<213>  Artificial

<220>
<223>  portion (I) ("carrier" or "trafficking" sequence)


<220>
<221>  misc_feature
<223>  Description of Sequence: NH2-RGSRR-COOH; (see page 4);

<220>
<221>  misc_feature
<222>  (1)..(5)
<223>  all amino acids are D-enantiomeric amino acids

<400>  22

Arg Gly Ser Arg Arg
1               5


<210>  23

<211>  4
<212>  PRT
<213>  Artificial

<220>
<223>  portion (I) ("carrier" or "trafficking" sequence)


<220>
<221>  misc_feature
<223>  Description of Sequence: NH2-RRQK-COOH; (see page 4);

<400>  23

Arg Arg Gln Lys
1


<210>  24
<211>  5
<212>  PRT
<213>  Artificial

<220>
<223>  portion (I) ("carrier" or "trafficking" sequence)


<220>
<221>  misc_feature
<223>  Description of Sequence: NH2-RARKG-COOH; (see page 4);

<220>
<221>  misc_feature
<222>  (1)..(5)
<223>  all amino acids are D-enantiomeric amino acids

<400>  24

Arg Ala Arg Lys Gly
1               5


<210>  25
<211>  4
<212>  PRT
<213>  Artificial

<220>
<223>  portion (I) ("carrier" or "trafficking" sequence)


<220>
<221>  misc_feature
<223>  Description of Sequence: NH2-RGRK-COOH; (see page 4);

<220>
<221>  misc_feature
<222>  (1)..(4)
<223>  all amino acids are D-enantiomeric amino acids

<400>  25

Arg Gly Arg Lys

1

<210> 26
<211> 5
<212> PRT
<213> Artificial

<220>
<223> portion (I) ("carrier" or "trafficking" sequence)

<220>
<221> misc_feature
<223> Description of Sequence: NH2-RRRLS-COOH; (see page 4);

<220>
<221> misc_feature
<222> (1)..(5)
<223> all amino acids are D-enantiomeric amino acids

<400> 26

Arg Arg Arg Leu Ser
1               5

<210> 27
<211> 7
<212> PRT
<213> Artificial

<220>
<223> portion (I) ("carrier" or "trafficking" sequence)

<220>
<221> misc_feature
<223> Description of Sequence: NH2-RPRRLSP-COOH; (see page 4);

<220>
<221> misc_feature
<222> (1)..(7)
<223> all amino acids are D-enantiomeric amino acids

<400> 27

Arg Pro Arg Arg Leu Ser Pro
1               5

<210> 28
<211> 5
<212> PRT
<213> Artificial

<220>
<223> portion (I) ("carrier" or "trafficking" sequence)

<220>
<221> misc_feature
<223> Description of Sequence: NH2-RGRKY-COOH; (see page 4);

<220>
<221>  misc_feature
<222>  (1)..(5)
<223>  all amino acids are D-enantiomeric amino acids

<400>  28

Arg Gly Arg Lys Tyr
1               5


<210>  29
<211>  7
<212>  PRT
<213>  Artificial

<220>
<223>  portion (I) ("carrier" or "trafficking" sequence)


<220>
<221>  misc_feature
<223>  Description of Sequence: NH2-RPKRGMG-COOH; (see page 4);

<220>
<221>  misc_feature
<222>  (1)..(7)
<223>  all amino acids are D-enantiomeric amino acids

<400>  29

Arg Pro Lys Arg Gly Met Gly
1               5


<210>  30
<211>  5
<212>  PRT
<213>  Artificial

<220>
<223>  portion (I) ("carrier" or "trafficking" sequence)


<220>
<221>  misc_feature
<223>  Description of Sequence: NH2-GVRRR-COOH; (see page 4);

<220>
<221>  misc_feature
<222>  (1)..(5)
<223>  all amino acids are D-enantiomeric amino acids

<400>  30

Gly Val Arg Arg Arg
1               5


<210>  31
<211>  9
<212>  PRT

<213>  Artificial

<220>
<223>  portion (I) ("carrier" or "trafficking" sequence)


<220>
<221>  misc_feature
<223>  Description of Sequence: NH2-GYKKVGFSR-COOH; (see page 4);

<220>
<221>  misc_feature
<222>  (1)..(9)
<223>  all amino acids are D-enantiomeric amino acids

<400>  31

Gly Tyr Lys Lys Val Gly Phe Ser Arg
1               5


<210>  32
<211>  7
<212>  PRT
<213>  Artificial

<220>
<223>  portion (I) ("carrier" or "trafficking" sequence)


<220>
<221>  misc_feature
<223>  Description of Sequence: NH2-KFSRLSK-COOH; (see page 4);

<220>
<221>  misc_feature
<222>  (1)..(7)
<223>  all amino acids are D-enantiomeric amino acids

<400>  32

Lys Phe Ser Arg Leu Ser Lys
1               5


<210>  33
<211>  4
<212>  PRT
<213>  Artificial

<220>
<223>  portion (I) ("carrier" or "trafficking" sequence)


<220>
<221>  misc_feature
<223>  Description of Sequence: NH2-RRVR-COOH; (see page 4);

<220>
<221>  misc_feature
<222>  (1)..(4)
<223>  all amino acids are D-enantiomeric amino acids

<400>  33

Arg Arg Val Arg
1


<210>  34
<211>  5
<212>  PRT
<213>  Artificial

<220>
<223>  portion (I) ("carrier" or "trafficking" sequence)


<220>
<221>  misc_feature
<223>  Description of Sequence: NH2-RRSRP-COOH; (see page 4);

<400>  34

Arg Arg Ser Arg Pro
1               5


<210>  35
<211>  4
<212>  PRT
<213>  Artificial

<220>
<223>  portion (I) ("carrier" or "trafficking" sequence)


<220>
<221>  misc_feature
<223>  Description of Sequence: NH2-RRRM-COOH; (see page 4);

<220>
<221>  misc_feature
<222>  (1)..(4)
<223>  all amino acids are D-enantiomeric amino acids

<400>  35

Arg Arg Arg Met
1


<210>  36
<211>  11
<212>  PRT
<213>  Artificial

<220>
<223>  portion (I) ("carrier" or "trafficking" sequence)


<220>
<221>  misc_feature
<223>  Description of Sequence: NH2-KSMALTRKGGY-COOH; (see page 4)

<220>

<221>  misc_feature
<222>  (1)..(11)
<223>  all amino acids are D-enantiomeric amino acids

<400>  36

Lys Ser Met Ala Leu Thr Arg Lys Gly Gly Tyr
1               5                   10


<210>  37
<211>  5
<212>  PRT
<213>  Artificial

<220>
<223>  portion (I) ("carrier" or "trafficking" sequence)


<220>
<221>  misc_feature
<223>  Description of Sequence: NH2-RSRRG-COOH; (see page 4);

<220>
<221>  misc_feature
<222>  (1)..(5)
<223>  all amino acids are D-enantiomeric amino acids

<400>  37

Arg Ser Arg Arg Gly
1               5


<210>  38
<211>  10
<212>  PRT
<213>  Artificial

<220>
<223>  Portion (I) ("carrier" or "trafficking" sequence)


<220>
<221>  misc_feature
<223>  Description of Sequence: NH2-RRRQRRKKRG-COOH; (see page 5);

<220>
<221>  misc_feature
<222>  (1)..(10)
<223>  all amino acids are D-enantiomeric amino acids

<400>  38

Arg Arg Arg Gln Arg Arg Lys Lys Arg Gly
1               5                   10


<210>  39
<211>  9
<212>  PRT
<213>  Artificial

<220>
<223>  Portion (I) ("carrier" or "trafficking" sequence)


<220>
<221>  misc_feature
<223>  Description of Sequence: NH2-RRRQRRKKR-COOH; (see page 5);

<220>
<221>  misc_feature
<222>  (1)..(9)
<223>  all amino acids are D-enantiomeric amino acids

<400>  39

Arg Arg Arg Gln Arg Arg Lys Lys Arg
1               5


<210>  40
<211>  8
<212>  PRT
<213>  Artificial

<220>
<223>  Portion (I) ("carrier" or "trafficking" sequence)


<220>
<221>  misc_feature
<223>  Description of Sequence: NH2-RRQRRKKR-COOH; (see page 5);

<220>
<221>  misc_feature
<222>  (1)..(8)
<223>  all amino acids are D-enantiomeric amino acids

<400>  40

Arg Arg Gln Arg Arg Lys Lys Arg
1               5


<210>  41
<211>  8
<212>  PRT
<213>  Artificial

<220>
<223>  Portion (I) ("carrier" or "trafficking" sequence)


<220>
<221>  misc_feature
<223>  Description of Sequence: NH2-RRRQRRKK-COOH; (see page 5);

<220>
<221>  misc_feature
<222>  (1)..(8)
<223>  all amino acids are D-enantiomeric amino acids

<400>  41

Arg Arg Arg Gln Arg Arg Lys Lys
1               5


<210>  42
<211>  7
<212>  PRT
<213>  Artificial

<220>
<223>  Portion (I) ("carrier" or "trafficking" sequence)


<220>
<221>  misc_feature
<223>  Description of Sequence: NH2-RQRRKKR-COOH; (see page 5);

<220>
<221>  misc_feature
<222>  (1)..(7)
<223>  all amino acids are D-enantiomeric amino acids

<400>  42

Arg Gln Arg Arg Lys Lys Arg
1               5


<210>  43
<211>  7
<212>  PRT
<213>  Artificial

<220>
<223>  Portion (I) ("carrier" or "trafficking" sequence)


<220>
<221>  misc_feature
<223>  Description of Sequence: NH2-RRQRRKK-COOH; (see page 5);

<220>
<221>  misc_feature
<222>  (1)..(7)
<223>  all amino acids are D-enantiomeric amino acids

<400>  43

Arg Arg Gln Arg Arg Lys Lys
1               5


<210>  44
<211>  6
<212>  PRT
<213>  Artificial

<220>
<223>  Portion (I) ("carrier" or "trafficking" sequence)


<220>
<221>  misc_feature

<223>  Description of Sequence: NH2-RRQRRK-COOH; (see page 5);

<220>
<221>  misc_feature
<222>  (1)..(6)
<223>  all amino acids are D-enantiomeric amino acids

<400>  44

Arg Arg Gln Arg Arg Lys
1               5


<210>  45
<211>  11
<212>  PRT
<213>  Artificial

<220>
<223>  Portion (I) ("carrier" or "trafficking" sequence)


<220>
<221>  MISC_FEATURE
<223>  Description of sequence: DM-G-DR-DR-DR-DQ-Dr-Dr-DK-DK-DR; (see
       page 31);

<220>
<221>  misc_feature
<222>  (1)..(1)
<223>  amino acid is D-enantiomeric amino acid

<220>
<221>  misc_feature
<222>  (2)..(2)
<223>  amino acid is D- or L-enantiomeric amino acid

<220>
<221>  misc_feature
<222>  (3)..(11)
<223>  all amino acids are D-enantiomeric amino acids

<400>  45

Met Gly Arg Arg Arg Gln Arg Arg Lys Lys Arg
1               5                   10

## Description

The present invention relates to novel conjugate molecules and their use. More particularly, the present invention relates to conjugate molecules containing a carrier and a cargo moiety, which are covalenly bound. A D amino acid sequence is covalently bound to specific drug molecules to act as a drug carrier, particularly for efficient intracellular delivery of anti-cancer and protease inhibitor drugs. The novel conjugate molecules are provided for the manufacture of a medicament for the treatment of e.g. cancer diseases.

It is undisputed that advances in pharmaceuticals have revolutionized health care for humans and other animals as well. However, despite the outstanding advances made in the field of pharmacology, some significant limitations still remain in the treatment of various diseases via drug agents. One of the most significant limitations at this time relates to the delivery of particular drugs in vivo, especially in situations where drugs are poorly water soluble or do not enter the cell through the hydrophobic cell membrane, e.g. due to their polar chemical structure. Indeed, the use of some drugs which show great promise in vitro, has been severely limited due to issues related to their water solubility or their insufficient cell permeability properties. While, in general, compounds showing a strong hydrophobicity profile are difficult to be administered via body liquids, compounds with strong polar properties do not cross the cell membrane. This causes problems with drug delivery in vivo. Cisplatin (cis-diamindichloroplatin (II)) (for the treatment of tumors, especially for example, in the case of bone cancer, bludder, lung or ovarian cancer) is an example of a huge number of water soluble drugs, which are not efficiently transported into the cell. In summary, it is an issue for the development of pharmaceuticals to ensure that the drug compound acting intracellularly is efficiently transported into the cytoplasm of cells and retained in the cell, if administered parenterally or non parenterally.

U.S. 4,675,381 discloses a polyaspartate and/or polyglutamate polymer as a drug carrier. This patent envisions the use of polyaspartate and/or polyglutamate polymers as drug carriers wherein the drug is encapsulated or incorporated in the matrix of the polymer. However, covalent linkage between the drug and the polymer to form conjugates is not envisaged therein. U.S. 5,087,616 discloses the use of a biodegradable polymeric carrier to which one or more cytotoxic molecules, for instance, daunomycin is conjugated. The biodegradable polymeric carrier is specified to be, for example, a homopolymer of polyglutamic acid. However, the use of a drug conjugated to polyglutamic acid does not enhance the cell uptake of the drug component by the cell.

A number of articles have been published addressing the physiological behaviour of anti-tumor drugs coupled to a homopolymer of polyaspartic acid (1982 Int. J. Cancer, Zunino et al.), to Poly(L-Glutamic Acid) ((1998) Cancer Research, Li et al.), to poly-amino acids, including polyaspartic acid ((1989 J. Pharm. Exp. Ther., Ramsammy), or to copolypeptides consisting of L-aspartic acid and L-glutamic acid ((1990) Biopolymers, Hayashi and Iwatsuki). Finally, U.S. 4,960,790 discloses the anti-tumor agent taxol covalently conjugated with, for example, an amino acid (for example, glutamic acid). All of these conjugates were synthesized to reduce the toxicity of the drug component or to improve its water solubility properties. The afore-mentioned prior art disclosure does not solve, however, the problem how to improve cell permeability of cargo molecules. In WO 94/044686 conjugate molecules containing a fragment of the HIV tat protein being covalently attached to macromolecular cargo molecules are disclosed. It is shown in WO 94/044686 that certain fragments of the HIV tat protein, e.g. from AA 37 to AA 58 of TAT protein, may enhance the cytoplasmatic delivery of macromolecules, e.g. proteins or nucleic acids. However, the improvement of cytoplasmatic delivery of the cargo molecules has turned out to be unsatisfying.

Moreover, WO 94/044686 did not provide any tool to transport small molecule anti-cancer drugs across the cell membrane into the cell. Thereby, it is to be noted that many small mole-cules used as anti-cancer drugs enter living cells at very low rate. As a consequence, strong efforts in the pharmaceutical industry have been made to improve the cellular uptake of e.g anti-cancer drugs not inherently capable of entering target cells at a useful rate or not entering the target cell at all. As indicated from the above art, there has been a long-felt need in the art to attempt to provide methods for efficient delivery of biologically active anti-tumor agents into the cells and retention of the same in the cell. The object of the present invention is to solve this object by the subject-matter as disclosed herein.

Accordingly, the present invention provides conjugate molecules which comprise at least one first portion (I) as a carrier moiety and at least one second portion (II) as a cargo moiety. Portion (II) is selected from a group of small molecule drugs, in particular anti-cancer drugs or protease inhibitors, whereas portion (I) is a peptide sequence containing at least three D-enantiomeric amino acids as defined by general formulae (a), (b), (c), (d), (e), (f), (g), (h), (i) as disclosed below.

In order to demonstrate one embodiment of the present invention, a conjugate of the anti-tumor agent Cisplatin was combined with a carrier moiety used as a drug delivery vehicle. It was then shown that this inventive conjugate possessed superior biological properties over, for example, unconjugated drugs. Experimental data shown below document that, for example, conjugating the antitumor drug Cisplatin (cargo moiety) to an inventive carrier moiety (portion (I)) results in unexpected enhancement of cellular drug uptake for drug resistant cell lines and longer drug retention in the cell.

The inventive conjugate molecule comprises as portion (I) ("carrier" or "trafficking" sequence") at least three contiguous D-enantiomeric amino acids, whereby the D amino acids are preferably selected from arginine or lysine residues. Therefore, functionally effective amino sequences as comprised by portion (I) and acting as carrier moieties show strong basic properties. In a preferred embodiment portion (I) comprises a peptide of the general formula (a) NH2-Xm-COOH, whereby X is selected from D amino acids arginine or lysine and "m" is an integer between 3 and 40, preferably 3 and 20 and most preferably between 3 and 12.

Another preferred functionally effective portion (I) of the inventive conjugate molecule comprises a sequence as defined by general formula (b) NH₂-XnArXn-COOH, wherein "X" relates to D-amino acid arginine or lysine, "A" relates to any non-basic D-amino acid and "n" and "r" represent an integer from 1 to 20, preferably from 3 to 10 and more preferably from 3 to 6 amino acids. Depending on the number of flanking "X" residues, "A" residue(s) may be positioned at any intrasequential position. Additional non-basic D-amino acids may be incorporated into the sequence as comprised by portion (I). Accordingly, functionally effective portions (I) of the inventive conjugate molecule may comprise a generic amino acid sequence of the following general formulae:
(c) NH₂-XpAoXpAoXp-COOH, (d) NH₂-AoXpAoXpAo-COOH, (e) NH₂₋XpAoXpAoXpAo-COOH, or (f) NH₂-AoXpAoXpAoXpAoXpAo-COOH, or (g) NH₂₋AoXpAoXpAoXpAoXpAoXpAo-COOH, (h) NH₂-AoXpAoXpAoXpAoXpAoXpAoXpAo-COOH, or (i) NH₂-AoXpAoXpAoXpAoXpAoXpAoXpAoXpAo-COOH, wherein "X" relates to D amino acids arginine or lysine, "A" relates to any non-basic D-amino acid, "o" is an integer from zero to fourteen, "p" is an integer, independent from "o", from 0 to 14.

Specific examples for functionally effective portions (I) of the inventive conjugate molecule contain or may consist of the following sequences showing strong basic properties: NH₂-KTRR-COOH, NH₂-RLKR-COOH, NH₂-KPRR-COOH, NH₂-KRFQR-COOH, NH₂-GRIRR-COOH, NH₂-NIGRRRN-COOH, NH₂-RAGRNGR-COOH, NH₂-RPRR-COOH, NH₂-GKRRG-COOH, NH₂-KRRE-COOH, NH₂-RQKRGGS-COOH, NH₂₋RKSR-COOH, NH₂-RGSRR-COOH, NH₂-RRQK-COOH, NH₂-RARKG-COOH, NH₂₋RGRK-COOH, NH₂-RRRLS-COOH, NH₂-RPRRLSP-COOH, NH₂-RGRKY-COOH, NH₂-RPKRGMG-COOH, NH₂-GVRRR-COOH, NH₂-GYKKVGFSR-COOH, NH₂₋KFSRLSK-COOH, NH₂-RRVR-COOH, NH₂-RRSRP-COOH, NH₂-RRRM-COOH, NH₂₋KSMALTRKGGY-COOH, NH₂-RSRRG-COOH (one-letter-code), whereby according to the invention, all amino acids are D-enantiomeric amino acids.

In a preferred embodiment, a trafficking sequence according to the invention can be derived, e.g., from a known membrane-translocating sequence of naturally occurring proteins. In a particularly preferred embodiment of the invention, the trafficking sequence of the conjugate molecule of the invention is a D-enantiomeric amino acid sequence in retro-inverso order of HIV TAT protein of human immunodeficiency virus (HIV). TAT is a viral protein indispensable for the HIV infection cycle. This protein is described in, e.g., WO 94/04686, U.S. Pat. Nos. 5,804,604 and 5,674,980, each incorporated herein by reference. According to the invention, portion (I) of the inventive conjugate molecule linked to its cargo portion (II) comprises some or all 86 amino acids (in its retro-inverso D-form) of the entire sequence that make up the TAT protein. In particular, a functionally effective portion (I) as carrier sequence of the inventive conjugate molecule has fewer than 86 D-amino acids still exhibiting uptake activity into cells and optionally uptake into the cell nucleus. Retro-inverso Tat sequences (composed of D-amino acids) including the region that mediates entry and uptake into cells can be defined using known techniques (see, e.g., Franked et al., Proc. Natl. Acad. Sci, USA 86: 7397-7401(1989). Preferably, portion (I) of the conjugate molecule of the invention comprises the basic region (amino acids 48-57 or 49-57, respectively) of TAT (hereinafter: D-Tat sequences) and does not comprise TAT's cysteine-rich region (amino acids 22-36) as well as the exon 2-encoded carboxy-terminal domain (amino acids 73-86) of the naturally-occuring TAT protein. Preferred peptidic sequences as comprised by portion (I) and being designed according to the native L amino acid sequence (AA48-AA57 of Tat) are e.g. (shown in their retro-inverso order (as compared to the naturally occurring sequence)) NH₂-RRRQRRKKRG-COOH or NH₂-RRRQRRKKR-COOH, or NH2-RRQRRKKR-COOH or NH2-RRRQRRKK-COOH or NH2-RQRRKKR-COOH, NH2-RRQRRKK-COOH or NH2-RRQRRK-COOH (all of them composed of D-amino acids). For any of the above given sequences from 1 to 5 arginine or lysine, respectively, residue(s) may be substituted by lysine or by arginine residue(s), respectively.

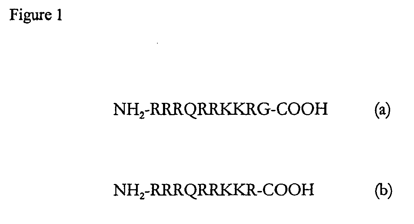

In a particularly preferred embodiment of the invention portion I comprises a carrier sequences of **figure 1** (D-Tat sequences).

The term "retro-inverso" relates to an isomer of a linear peptide in which the direction of the sequence is reversed and the chirality of each amino acid residue is inverted. Retro-inverso conjugate molecules according to the invention can be constructed, e.g., by synthesizing a reverse of the amino acid sequence for the corresponding native L-amino acid sequence. In D-retro-inverso enantiomeric peptides as comprised by portion (I) the positions of carbonyl and amino groups in each single amide bond are exchanged, while the position of the side-chain groups at each alpha carbon is preserved. Retro-inverso peptides as used for inventive conjugate molecules possess a variety of useful properties. For example, they enter cells more efficiently and are more stable (especially *in vivo)* and show lower immunogenicity than corresponding L-peptides. Naturally-occuring proteins contain L-amino acids. Therefore, almost all decomposition enzymes, like proteases or peptidases, cleave peptide bonds between adjacent L-amino acids. Consequently, peptides composed of D-enantiomeric amino acids in retro-inverso form are largely resistant to proteolytic breakdown.

Portion (I) of the inventive conjugate molecule serves as a carrier or trafficking sequence. A "trafficking or carrier sequence" is any sequence of amino acids that directs a conjugate molecule or portion (II) of the conjugate molecule, respectively, into the cell cytoplasm or, even further, to a specific cellular destination. The trafficking sequence can e.g. direct the conjugate molecule to a desired location within the cell, e.g., the nucleus, the ribosome, the endoplasmatic reticulum, a lysosome, or a peroxisome. Consequently, in a preferred embodiment the trafficking sequence of the conjugate molecule of the invention directs the conjugate molecule to a defined cellular location. Anyhow, the trafficking sequence can direct the inventive conjugate molecule across the plasma membrane, e.g., from the extracellular cell environment through the plasma membrane into the cytoplasma thereby enhancing the cellular uptake of the conjugate molecule or its drug portion (II) (cargo portion), respectively, in particular by enhancing its cell permeability or by enhancing its intracellular retention time.

Functionally effective portions (I) of the inventive conjugate molecule preferably comprise an amino acid sequence as translocation sequence with a length ranging from 3 to 50 D-amino acids, more preferably from 4 to 40 D-amino acids, even more preferably from 4 to 30 D-amino acids, even more preferably from 4 to 20 D-amino acids and most preferably from 4 to 12 D-amino acids. Preferably, portion (I) of an inventive conjugate molecule comprises a functionally effective translocation sequence containing at least 60%, preferably at least 65%, more preferably at least 70%, even more preferably at least 75%, most preferably at least 80% or at least 90 % basic amino acids, preferably arginine and/or lysine residues. If the translocation sequence of portion (I) has from 4 to 12 D-amino acids, its basic amino acid number ranges from 2 to 12. Retro-inverso forms of portion (I) according to the invention can be obtained from the corresponding (naturally occurring) peptide sequence (composed of L-amino acids) by synthetisizing a reverse of the amino acid sequence of the L-form by e.g. solid phase synthesis using D-amino acids.

Portion (I) may comprise just one (i.e., continuous) basic cell membrane translocation sequence (D-retro-inverso form) analogous to the corresponding (naturally-occuring) sequence, e.g. a fragment of the Tat sequences as disclosed above. Alternatively, portion (I) may also comprise two or more amino acid sequences in retro-inverso order which correspond to identical or different translocation sequences with strong basic properties, eventually synthesized on the basis of naturally-occurring protein(s), in other words, a combination of two (naturally occurring) translocation sequences which combination does not occur in any native protein sequence. These two or more translocation sequences of portion (I) may be linked together with or without a linker sequence. If a linker sequence is desired, its length will preferably range from 2 to 20 amino acids. The desired L-amino acid peptide sequence (as a starting material for the following synthesis of retro-inverso D peptidic sequences as used for portion (I) of the inventive conjugate molecule) can be obtained from naturally-occurring sources or can be produced by using genetic engineering techniques or by chemical synthesis.

As disclosed above, portion (I) may contain (one or more) D-enantiomeric amino acid translocation motifs (in retro-inverso order as compared to the native sequence), which are based on the corresponding naturally-occurring protein(s) or protein fragment(s)). Alternatively, portion (I) may contain D amino acid strings (in retro-inverso order) of functional equivalents of the naturally occurring protein (fragment) sequences (also called "derivatives" herein). These functional equivalents (according to the invention being contained in portion (I) in retro-inverso order) still possess carrier properties with uptake activity into the cell (or even, preferably, into the cell nucleus) that is substantially similar to that of the corresponding naturally-occurring protein, even though their sequence is not identical with the naturally occurring protein sequence or a fragment thereof.

To produce derivatives, the amino acid sequence of equivalents of naturally-occurring proteins or rather their translocation sequence (e.g., TAT's translocation sequence), and thus, of their inventive retro-inverso forms (portion (I)) comprising D-enantiomeric amino acids (e.g., D-TAT) can be provided on the basis of modifications of the native sequence, for example, by addition, deletion and/or substitution of at least one amino acid present of the naturally-occurring protein, to produce modified starting material for the synthesis of retro-inverso portion (I). Portions (I) based on modified translocation sequence(s) with increased or decreased stability can be produced using known techniques (see below, definition of "derivatives"). In addition, sugar moieties and/or lipid moieties, e.g. cholesterol or other lipids, may be added to the peptides used as portion (I) of the inventive conjugate molecule in order to further increase the membrane solubility of the conjugate molecule, e.g. to one or both termini of portion (I) to provide local lipophilicity at one or both termini. Alternatively or additionally, sugar or lipid moieties may be linked to the D amino acid side chains, in particular side chains having terminal hydroxyl and amino groups.

Portion (I) of the inventive conjugate molecule has to retain its cell permeability an/or its intracellular retention function. However, other functions may be added by modifications introduced into portion (I). Therefore, portion (I) can be modified, e.g., to efficiently direct the conjugate molecule of the invention to a particular intracellular target localization. Correspondingly, portion (I) is modified such that a specific intracellular localization is awarded to portion (I) without loss of its enhanced cell permeability properties. Typically, a routing sequence for targeting the inventive conjugate molecule to specific cell compartments (e.g., endoplasmic reticulum, mitochondrion, gloom apparatus, lysosomal vesicles) can be introduced into portion (I). On the other hand, specific sequences which ensure cytoplasmatic localization of the inventive conjugate molecule may be added. E.g., portion (I) may comprise at least one further sequence which binds to one or more cytoplasmatic structure(s) in order to retain the conjugate molecule of the invention in the cytoplasm. Alternatively, alteration of the basic region thought to be important for nuclear localization (see, e.g., Dang and Lee (1989), J.Biol.Chem. 264:18019-18023; Hauber et al. (1989), J.Virol. 63:1181-1187; Ruben et al. (1989), J.Virol. 63:1-8) can result in a cytoplasmic location or partially cytoplasmic location of portion (I), and, therefore, of the conjugate molecule of the invention. Therefore, portion (I) may contain altered nuclear localization signals, which lead to cytoplasmatic localization of the inventive conjugate molecule.

Portion (II) of the inventive conjugate molecule represents the biologically active cargo moiety. Portion (II) preferably contains an anti-tumor drug, in particular alkylating drugs, antimetabolica, cytostatics or drugs related to hormone treatment. It is preferred to select as anti-tumor drugs compounds of the platin (derivative) and taxol classes. In particular, the drug moiety is selected from the group of drugs consisting of, for example, cisplatin, satraplatin, oxaliplatin, carboplatin, nedaplatin, chlorambucil, cyclophosphamid, mephalan, azathioprin, fluorouracil, mercaptopurin, methrexat, nandrolon, aminogluthemid, medroxyprogesteron, megestrolacetate, procarbazin, docetaxel, paclitaxel, epipodophyllotoxin, podophyllotoxin, vincristine, docetaxel, daunomycin, doxorubicin, mitoxantrone, topotecan, bleomycin, gem-citabine, fludarabine, and 5-FUDR.

Alternatively, portion (II) comprises protease inhibitors, i.e. drug molecules, which inhibit proteases, in particular proteases which are involved in the infection cycle of infectious agents, e.g. viral, bacterial or protozoological proteases. In a preferred embodiment, these protease inhibitors as part of an inventive conjugate molecule may serve to treat viral, bacterial infections or protozoological infections, e.g. malaria. In particular, virus infections may be treated by protease inhibitors, e.g. retroviral diseases. The use of conjugate molecules comprising protease inhibitors are strongly preferred for the treatment of HIV infections. The protease inhibitors to be used for coupling to carrier sequence as disclosed herein may be selected from a group containing the 640385, abacavir sulfate, AG1776, amprenavir (141W94 or VX-478), atazanavir ( BMS-232632), Cathepsin S protease inhibitor, D1927, D9120, efavirenz, emtricitabine, enfuvirtide (T-20), fosamprenavir (GW-433908 or VX-175), GS 9005, GW640385 (VX-385), HCV protease inhibitor, indinavir (MK-639), L-756, 423, levoprin-ZG, lopinavir (ABT-378), lopinavir/ritonavir (LPV ABT-378/r), MK-944A, mozenavir (DMP450), nelfinavir (AG-1343), nevirapine, P-1946, PL-100, prinomastat, ritonavir (ABT-538), RO033-4649, TMC114, saquinavir (Ro-31-8959), tenofovir disoproxil fumarate, tipranavir (PNU-140690), TLK 19781, TMC-114, Vertex 385, VX-950.

In a further preferred embodiment, at least two drug moieties, which may or may not be the same, are combined in the inventive conjugate molecule. If more than one drug molecule is contained in the inventive conjugate molecule, e.g. two cisplatin molecules or a combination of a cisplatin and a satraplatin molecule, these drug moieties can both be linked together, eventually via spacer or linker groups, and coupled a such (one single cargo complex built of two (or more) drug molecules) to portion (I) or, alternatively, may be coupled to portion (I) of the inventive conjugate molecule independently upon each other. E.g. they may be linked on either terminus of portion (I), e.g. to the terminal amino and carboxyl group and/or to suitable side chain groups of D amino acids of portion (I). Accordingly, the inventive conjugate molecule may comprise a plurality of drug moieties, coupled to portion (I) as one single complex portion (II) or coupled to portion (I) separately (giving more than one portion (II).

The drug molecule(s) of portion(s) (II) typically make up from 10 percent to 60 percent, by weight, more preferably from 10 percent to 50 percent, by weight, and most preferably from 10 percent to 40 percent, by weight of the inventive conjugate molecule. Correspondingly, the carrier moiety of portion (I) may make up from 40 percent to 90 percent, by weight, more preferably from 50 percent to 90 percent, by weight, and most preferably from 60 percent to 90 percent, by weight of the inventive conjugate molecule. The molecular weight of the inventive conjugate molecule is typically from about 1,000 to about 50,000 Dalton, preferably from 1,000 to 20,000 and more preferably from 1,000 to 5,000.

Conjugate molecules of the invention are composed at least of portion (I) and at least of one portion (II). Moreover, the conjugate molecule according to the invention may comprise further portions (III), (IV) or (V). These additional portions are optional and may award additional functions to the inventive conjugate molecule. The at least one further portion can be an amino acid, oligopeptide, a sugar moiety, e.g. a complex sugar chain, a lipid or a polypeptide or an small organo-chemical compound and can be linked to the conjugate molecule of the invention at a suitable position, for example, the N-terminus, the C-terminus or internally. Such further portions (e.g, HA, HSV-Tag, His6) may render the inventive conjugate molecule amenable to purification and/or isolation. If desired, the fusion partner can then be removed from conjugate molecule of the invention (e.g., by proteolytic cleavage or other methods known in the art) at the end of the production process. Alternatively, further trafficking sequences for specific cell compartments or other functional sequences may be fused to the inventive molecule by an additional portion or may be incorporated into portion (I) or (II). Preferably, an additional portion (e.g. portion (III)) allows the inventive conjugate molecule to specifically bind to a certain cell type, e.g immune cells, hepatocytes etc. This object is achieved by fusing naturally occurring ligands (e.g. ligands for extracellular portions of membrane proteins, like receptors, or antibodies directed to extracellular portions of membrane proteins) for certain cell markers to the inventive conjugate molecule, e.g. to bind to a target tumor cell. Thereby, the inventive conjugate molecule may be directed selectively to certain cells or tissues of an animal to be treated.

In an preferred embodiment of the invention, the at least one first portion (I) and the at least one second portion (II) of the conjugate molecule of the invention are linked by a covalent bond. "Covalent bond" relates to a stable chemical link between two atoms produced by sharing one or more pairs of electrons. If present, further portions (III), (IV), (V) etc., as mentioned above, can also be linked by a covalent bond to the inventive conjugate molecule, preferably to its portion (I).

In general, portion (I) and portion (II) can be coupled via a linker or directly (without linker) by e.g. an amide bridge, if the portions to be linked have reactive amino or carboxy groups. Alternatively, ester or ether linkages are preferred.

If present, further portions (III), (IV), (V) etc., as mentioned above, can be coupled in an analogous manner to portion (I) and/or portion (II) or, optionally, with each other to then be linked as one single moiety to either portion (I) or portion(s) (II). Linker sequences can also be used to fuse the conjugate molecule of the invention with at least one other portion (see below). The mode of coupling further portion(s) to the either portion (I) or portion (II) of the inventive conjugate molecule will depend on its chemical character. If additional portions (III), (IV) etc. belong to the class of peptidic sequences, they will preferably linked to the inventive conjugate molecule to either terminus of portion (I) or, alternatively, be linked via portion (I)'s D amino acid side chains, e.g. by a disulfide bridge. Further portions of other chemical nature may be likewise attached to portion (I) (terminal groups or chemically active side chain groups) or portion (II). The linkage via a side chain will preferably be based on side chain amino, thiol or hydroxyl groups, e.g. via an ester or ether linkage. It has to be noted that, according to the invention, all amino acids (of any of portions (I), and, if built of amino acids, portions (III), (IV), (V) etc.,) are D-enantiomeric amino acids, which reflect its eventually naturally occurring analogue by being linked in retro-inverso order.

If peptidic linker sequences are used to fuse portion (I) and (II) or to fuse another portion, e.g. (III) to portion (I) and/or (II), the linker sequences preferably form a flexible sequence of 5 to 50 residues, more preferably 5 to 15 residues. In a preferred embodiment the linker sequence contains at least 20%, more preferably at least 40% and even more preferably at least 50% Gly residues. Appropriate linker sequences can be easily selected and prepared by a person skilled in the art

Preferably, portion (I) and portion are linked by chemical coupling in any suitable manner known in the art. However, attention is drawn to the fact that many known chemical cross-linking methods are non-specific, i.e., they do not direct the point of coupling to any particular site on the carrier moiety or cargo moiety. Thus, the use of non-specific cross-linking agents may attack functional sites or sterically block active sites, rendering the fused portions of the inventive conjugate molecule biologically inactive. It is referred to the knowledge of the skilled artisan to block potentially reactice groups by using appropriate protecting groups. If present, further portions (III), (IV), (V) etc., as mentioned above, can be chemically coupled in an analogous manner to one another or to portion (I) and/or (II).

Coupling specificity can be increased by direct chemical coupling to a functional group found only once or a few times in portion (I), which functional group is to be cross-linked to the organic molecule of portion (II). As an example, the cystein thiol group may be used, if just one cystein residue is present on portion (I) of the inventive conjugate molecule. Also, for example, if a conjugate molecule portion (I) contains no lysine residues, a cross-linking reagent specific for primary amines will be selective for the amino terminus of portion (I). However, if a cysteine residue is to be introduced into portion (I), introduction at or near its N- or C-terminus is preferred. Conventional methods are available for such amino acid sequence alterations based on modifications of portion (I) by either adding one or more additional amino acids, e.g. inter alia an cystein residue, to the translocation sequence or by substituting at least one residue of the translocation sequence(s) being comprised in portion (I). In case a cystein side chain is used for coupling purposes, portion (I) of the inventive conjugate molecule has preferably one cystein residue. Any second cystein residue should preferably be avoided and can, eventually, be replaced when they occur in portion (I) of the inventive conjugate molecule. When a cysteine residue is replaced in the original translocation sequence to be used as or as part of portion (I), it is typically desirable to minimize resulting changes in portion (I) peptide folding. Changes in portion (I) folding are minimized when the replacement is chemically and sterically similar to cysteine. Therefore, serine is preferred as a replacement for cysteine.

Coupling of the two constituents of the inventive conjugate molecule can be accomplished via a coupling or conjugating agent. There are several intermolecular cross-linking reagents which can be utilized, see for example, Means and Feeney, Chemical Modification of Proteins, Holden-Day, 1974, pp. 39-43. Among these reagents are, for example, N-succinimidyl 3-(2-pyridyldithio)propionate (SPDP) or N,N'-(1,3-phenylene)bismaleimide; N,N'-ethylene-bis-(iodoacetamide) or other such reagent having 6 to 11 carbon methylene bridges; and 1,5-difluoro-2,4-dinitrobenzene. Other cross-linking reagents useful for this purpose include: p,p'-difluoro-m,m'-dinitrodiphenylsulfone; dimethyl adipimidate; phenol-1,4-disulfonylchloride; hexamethylenediisocyanate or diisothiocyanate, or azophenyl-p-diisocyanate; glutaraldehyde and disdiazobenzidine. Cross-linking reagents may be homobifunctional, i.e., having two functional groups that undergo the same reaction. A preferred homobifunctional cross-linking reagent is bismaleimidohexane (BMH). BMH contains two maleimide functional groups, which react specifically with sulfhydryl-containing compounds under mild conditions (pH 6.5-7.7). The two maleimide groups are connected by a hydrocarbon chain. Therefore, BMH is useful for irreversible cross-linking of proteins (or polypeptides) that contain cysteine residues. Cross-linking reagents may also be heterobifunctional. Heterobifunctional cross-linking agents have two different functional groups, for example an amine-reactive group and a thiol-reactive group, that will cross-link two proteins having free amines and thiols, respectively. Examples of heterobifunctional cross-linking agents are succinimidyl 4-(N-maleimidomethyl) cyclohexane-1-carboxylate (SMCC), m-maleimidobenzoyl-N-hydroxysuccinimide ester (MBS), and succinimide 4-(p-maleimidophenyl)butyrate (SMPB), an extended chain analog of MBS. The succinimidyl group of these cross-linkers reacts with a primary amine, and the thiol-reactive maleimide forms a covalent bond with the thiol of a cysteine residue. Because cross-linking reagents often have low solubility in water, a hydrophilic moiety, such as a sulfonate group, may be added to the cross-linking reagent to improve its water solubility. Sulfo-MBS and sulfo-SMCC are examples of cross-linking reagents modified for water solubility. Many cross-linking reagents yield a conjugate that is essentially non-cleavable under cellular conditions. Therefore, some cross-linking reagents contain a covalent bond, such as a disulfide, that is cleavable under cellular conditions. For example, Traut's reagent, dithiobis (succinimidylpropionate) (DSP), and N-succinimidyl 3-(2-pyridyldithio)propionate (SPDP) are well-known cleavable cross-linkers. The use of a cleavable cross-linking reagent permits the cargo moiety to separate from the transport polypeptide after delivery into the target cell. For this purpose, direct disulfide linkage may also be useful. Chemical cross-linking may also include the use of spacer arms. Spacer arms provide intramolecular flexibility or adjust intramolecular distances between conjugated moieties and thereby may help preserve biological activity. A spacer arm may be in the form of a protein (or polypeptide) moiety that includes spacer amino acids, e.g. proline. Alternatively, a spacer arm may be part of the cross-linking reagent, such as in "long-chain SPDP" (Pierce Chem. Co., Rockford, Ill., cat. No. 21651 H). Numerous cross-linking reagents, including the ones discussed above, are commercially available. Detailed instructions for their use are readily available from the commercial suppliers. A general reference on protein cross-linking and conjugate preparation is: Wong, Chemistry of Protein Conjugation and Cross-Linking, CRC Press (1991).

The inventive conjugate molecule may comprise the retro-inverso D-form of at least one naturally-occurring translocation sequence. However, these naturally occurring translocation sequences may be modified ("derivatives" of naturally occuring (translocation) sequences). Consequently, the inventive conjugate molecule may comprise such derivatives in its peptidic portion(s), e.g. portion (I). Therefore, a "derivative" or "derivative of a conjugate molecule" according to the invention is intended to mean derivative of peptidic portions, e.g. portion (I) and/or eventually further peptidic portions of the inventive conjugate molecule). It is intended to indicate a conjugate molecule the peptidic portion(s) of which is/are derived from the naturally occurring matrix by way of substitution(s) of one or more amino acids at one or more of sites of the amino acid sequence, by way of deletion(s) of one or more amino acids at any site of the naturally occuring matrix sequence, and/or by way of insertion(s) of one or more amino acids at one or more sites of the naturally occuring peptide sequence. "Derivatives" shall retain their characteristic activity, if used as portion of the inventive conjugate molecule, e.g. a derivative of the translocation sequence of peptidic portion (I) has to retain its translocation efficieny. Derivatives have to be functionally homologous.

If substitution(s) of amino acid(s) are used for the preparation of a derivative of naturally occurring sequences, conservative substitutions are preferred. Conservative substitutions typically include substitutions within the following groups: glycine and alanine; valine, isoleucine and leucine; aspartic acid and glutamic acid; asparagine and glutamine; serine and threonine; lysine and arginine; and phenylalanine and tyrosine. Thus, preferred conservative substitution groups are aspartate-glutamate; asparagine-glutamine; valine-leucine-isoleucine; alanine-valine; phenylalanine-tyrosine and lysine-arginine. By such mutations of peptidic portions of the inventive conjugate molecule e.g. their stability and/or effectiveness can be enhanced. Peptidic portions of the inventive conjugate molecules having mutated sequences such that they remain homologous, e.g. in sequence, in function, and in antigenic character or other function, with a protein having the corresponding parent sequence are encompassed by the invention. It is particularly preferred that the derivatives of the trafficking sequence being comprised in portion (I) remain functional (maintain their character as cell permeable moiety). Such mutated peptidic portions of inventive conjugate molecules can possess altered properties which may be advantageous over the properties of the inventive sequence for certain applications (e.g. increased pH optimum, increased temperature stability etc.).

Since inventive conjugate molecules are composed of D-amino acids in retro-inverso order, a derivative as used for the inventive conjugate molecule is termed "D-form derivative". The D-form derivative is synthesized on the basis of the "L-from derivative", which directly reflects the modifications introduced as compared to the corresponding naturally-occuring amino acid sequence.

A derivative of peptidic portion(s) of inventive conjugate molecule is defined as to have substantial identity with the amino acid sequences of naturally occurring sequences, e.g. naturally occurring translocation sequences, e.g. with the HIV Tat protein translocation sequence. Particularly preferred are amino acid sequences which have at least 30% sequence identity, preferably at least 50% sequence identity, even preferably at least 60% sequence identity, even preferably at least 75% sequence identity, even more preferably at least 80%, yet more preferably 90% sequence identity and most preferably at least 95% sequence identity to the naturally occurring analogue. Appropriate methods for isolation of a functional derivative of a conjugate molecule as well as for determination of percent identity of two amino acid sequences are described below. Sequence identity can be measured, e.g., by using sequence analysis software (Sequence Analysis Software Package of the Genetics Computer Group, University of Wisconsin Biotechnology Center, 1710 University Avenue, Madison, Wis. 53705) with the default parameters therein.

The production of derivatives is well known and can be carried out following standard methods which are well known by a person skilled in the art (see e.g., Sambrook J, Maniatis T (1989) *supra).* In general, the preparation of derivatives can be achieved by modifying a DNA sequence which encodes the naturally-occuring L-form of the peptidic sequence used as a matrix for the D-peptidic portions of the conjugate molecule of the invention by transformation of that DNA sequence into a suitable host and expression of the modified DNA sequence to form the functional derivative of the L-amino acid peptide ("L-form derivative") with the provision that the modification of the DNA does not disturb the characteristic activity. The isolation of such L-form derivatives can be carried out using standard methods including separating the (host) cells from the medium by centrifugation or filtration, if necessary after disruption of the cells, precipitating the proteinaceous components of the supernatant or filtrate by means of a salt, e. g., ammonium sulfate, followed by purification by using a variety of chromatographic procedures, e. g., ion exchange chromatography, affinity chromatography or similar art recognized procedures (see, e.g., Sambrook J, Maniatis T (1989) *supra).* Subsequently, the D-enantiomeric retro-inverso peptidic portion of the invention can be produced by synthesizing the reverse amino acid sequence of said L-form derivative resulting in the D-form derivative as used for portion(s) of the inventive conjugate molecule.

As mentioned above, the conjugate molecules of the invention may be produced by synthesizing a reverse amino acid sequence of the corresponding naturally occurring L-form amino acid sequence. This synthesis is preferably carried out by solid phase synthesis linking D amino acids to the desired retro-inverso sequence. Apart from the D amino acids used and the synthesis of the amino acids in retro-inverso order the solid phase synthesis of the inventive D amino acid sequences is chemically identical with the synthesis of peptides on the basis of L amino acids.

The starting material (matrix for the retro-inverso D amino acid peptide) for the synthesis of the peptide as used as portion (I) of the inventive conjugate molecule may also be produced by recombinant methods. Recombinant methods are preferred if a high yield is desired. A general method for the construction of any desired DNA sequence is provided, e.g., in Brown J. et al. (1979), Methods in Enzymology, 68:109; Sambrook J, Maniatis T (1989), *supra.* Subsequently, the D-retro-inverso-enantiomeric portion (I) of the inventive conjugate molecule is synthesized as described above. Alternatively, the matrix for portion (I) of the invention can be produced by *in vitro* translation of a nucleic acid that encodes the naturally-occuring L-form of portion (I) of the inventive conjugate molecule, by chemical synthesis (e. g., solid phase protein synthesis) or by any other suitable method. Subsequently, the D-retro-inverso-enantiomeric form of the inventive conjugate molecule is synthesized as described above.

Efficient methods for producing the portion (I) or other peptidic D-amino acid portions of the conjugate molecule according to the present invention also include to utilize genetic engineering techniques by transforming a suitable host cell with a nucleic acid or a vector provided herein which encodes the L-form of the portion (I) of the inventive conjugate molecule and cultivating the resultant recombinant microorganism, preferably *E.coli,* under conditions suitable for host cell growth and nucleic acid expression, e.g., in the presence of inducer, suitable media supplemented with appropriate salts, growth factors, antibiotic, nutritional supplements, etc.), whereby the nucleic acid is expressed and the encoded portion (I) matrix peptide (containing L-amino acids) is produced. Subsequently, the D-retro-inverso-enantiomeric form is synthesized as described above.

A vector comprising the nucleic acid of the L-form of an peptidic portion, e.g. portion (I) of the inventive conjugate molecule defines a nucleic acid sequence which comprises one or more nucleic acid sequences of the L-form of a portion, e.g. portion (I) of the inventive conjugate molecule and, eventually, other sequences. A vector can be used, upon transformation into an appropriate host cell, to cause expression of said nucleic acid. The vector may be a plasmid, a phage particle or simply a potential genomic insert. Once transformed into a suitable host, the vector may replicate and function independently of the host genome, or may, under suitable conditions, integrate into the genome itself. Preferred vectors according to the invention are *E.coli* XL-Blue MRF' and pBK-CMV plasmid.

The afore-mentioned term "other sequences" of a vector relates to the following: In general, a suitable vector includes an origin of replication, for example, Ori p, colEl Ori, sequences which allow the inserted nucleic acid to be expressed (transcribed and/or translated) and/or a selectable genetic marker including, e.g., a gene coding for a fluorescence protein, like GFP, genes which confer resistance to antibiotics such as the p-lactamase gene from Tn3, the kanamycin-resistance gene from Tn903 or the chloramphenicol-resistance gene from Tn9.

The term "plasmid" means an extrachromosomal usually self-replicating genetic element. Plasmids are generally designated by a lower "p" preceded and/or followed by letters and numbers. The starting plasmids herein are either commercially available, publicly available on an unrestricted basis or can be constructed from available plasmids in accordance with the published procedures. In addition, equivalent plasmids to those described are known to a person skilled in the art. The starting plasmid employed to prepare a vector of the present invention may be isolated, for example, from the appropriate *E. coli* containing these plasmids using standard procedures such as cesium chloride DNA isolation.

A suitable vector also relates to a (recombinant) DNA cloning vector as well as to a (recombinant) expression vector. A DNA cloning vector refers to an autonomously replicating agent, including, but not limited to, plasmids and phages, comprising a DNA molecule to which one or more additional nucleic acids of the L-form of one or more portions of the inventive conjugate molecule have been added. An expression vector relates to any DNA cloning vector recombinant construct comprising a nucleic acid sequence of the L-form of one or more portion(s) of the inventive conjugate molecule operably linked to a suitable control sequence capable of effecting the expression and to control the transciption of the inserted nucleic acid in a suitable host. Such plasmids may also be readily modified to construct expression vectors that produce the L-form of peptidic portion(s) of the inventive conjugate molecule in a variety of organisms, including, for example, *E. coli,* Sf9 (as host for baculovirus), *Spodoptera* and *Saccharomyces.* The literature contains techniques for constructing AV12 expression vectors and for transforming AV12 host cells. U.S. Pat. No. 4,992,373, herein incorporated by reference, is one of many references describing these techniques.

"Operably linked" means that the nucleic acid sequence is linked to a control sequence in a manner which allows expression (e. g., transcription and/or translation) of the nucleic acid sequence. "Transcription" means the process whereby information contained in a nucleic acid sequence of DNA is transferred to complementary RNA sequence

"Control sequences" are well known in the art and are selected to express the nucleic acid of the L-form of the peptidic portion(s) and to control the transcription. Such control sequences include, but are not limited to a polyadenylation signal, a promoter (e.g., natural or synthetic promotor) or an enhancer to effect transcription, an optional operator sequence to control transcription, a locus control region or a silencer to allow a tissue-specific transcription, a sequence encoding suitable ribosome-binding sites on the mRNA, a sequence capable to stabilize the mRNA and sequences that control termination of transcription and translation. These control sequences can be modified, e.g., by deletion, addition, insertion or substitution of one or more nucleic acids, whereas saving their control function. Other suitable control sequences are well known in the art and are described, for example, in Goeddel (1990), Gene Expression Technology:Methods in Enzymology 185, Academic Press, San Diego, CA.

Especially a high number of different promoters for different organism is known. For example, a preferred promoter for vectors used in *Bacillus subtilis* is the AprE promoter; a preferred promoter used in *E. coli* is the T7/Lac promoter, a preferred promoter used in *Saccharomyces cerevisiae* is PGK1, a preferred promoter used in *Aspergillus niger* is glaA, and a preferred promoter used in *Trichoderma reesei (reesei)* is cbhI. Promoters suitable for use with prokaryotic hosts also include the beta-lactamase (vector pGX2907 (ATCC 39344) containing the repli-con and beta-lactamase gene) and lactose promoter systems (Chang et al. (1978), Nature (London), 275:615; Goeddel et al. (1979), Nature (London), 281:544), alkaline phosphatase, the tryptophan (trp) promoter system (vector pATH1 (ATCC 37695) designed to facilitate expression of an open reading frame as a trpE fusion protein under control of the trp promoter) and hybrid promoters such as the tac promoter (isolatable from plasmid pDR540 ATCC-37282). However, other functional bacterial promoters, whose nucleotide sequences are generally known, enable a person skilled in the art to ligate them to DNA encoding the L-forms of peptidic portion(s) of the present invention using linkers or adapters to supply any required restriction sites. Promoters for use in bacterial systems also will contain a Shine-Dalgamo sequence operably linked to the DNA encoding the desired L-form of peptidic portion(s) of the invention.

Useful expression vectors, for example, may consist of segments of chromosomal, non-chromosomal and synthetic DNA sequences such as various known derivatives or fragments of SV40 and known bacterial plasmids, e.g., plasmids from *E. coli* including col E1, pBK, pCR1, pBR322, pMb9, pUC 19 and their derivatives, wider host range plasmids, e.g., RP4, phage DNAs e.g., the numerous derivatives of phage lambda, e.g., NM989, and other DNA phages, e.g., M13 and filamentous single stranded DNA phages, yeast plasmids, vectors useful in eukaryotic cells, such as vectors useful in animal cells and vectors derived from combinations of plasmids and phage DNAs, such as plasmids which have been modified to employ phage DNA or other expression control sequences. Expression techniques using the expression vectors described above are known in the art and are described generally in, for example, Sambrook J, Maniatis T (1989) *supra.*

Suitable "cells" or "host cells" comprising an aforementioned vector or a nucleic acid of the L-form of the peptidic portion(s) of the inventive conjugate molecule have the capacity to act as a host and expression vehicle for a nucleic acid or a vector as described above. The host cell can be e.g., a prokaryotic, an eukaryotic or an archaeon cell. Host cells comprise (for example, as a result of transformation, transfection or tranduction) a vector or nucleic acid as described herein include, but are not limited to, bacterial cells (e.g., R. *marinus, E. coli, Streptomyces, Pseudomonas, Bacillus, Serratia marcescens, Salmonella typhimurium),* fungi including yeasts (e. g*., Saccharomycies cerevisie, Pichia pastoris)* and molds (e.g., *Aspergillus sp.),* insect cells (e.g., Sf9) or mammalian cells (e.g., COS, CHO). Preferably, host cells means the cells of *E*. *coli.* In general, a host cell may be selected modulating the expression of inserted sequences of interest or modifying or processing expressed proteins encoded by the sequences in the specific manner desired. Appropriate cells or cell lines or host systems may thus be chosen to ensure the desired modification and processing of the foreign protein is achieved. For example, protein expression within a bacterial system can be used to produce an unglycosylated core protein, whereas expression within mammalian cells ensures "native" glycosylation of a heterologous protein.

Eukaryotic host cells are not limited to use in a particular eukaryotic host cell. A variety of eukaryotic host cells are available, e.g., from depositories such as the American Type Culture Collection (ATCC) and are suitable for use with vectors as described above. The choice of a particular host cell depends to some extent on the particular expression vector used to drive expression of the nucleic acids of the L-form of the peptidic portion(s) of the invention. Eukaryotic host cells include mammalian cells as well as yeast cells. The imperfect fungus *Saccharomyces cerevisiae* is the most commonly used eukaryotic microorganism, although a number of other strains are commonly available. For expression in *Saccharomyces sp.,* the plasmid YRp7 (ATCC-40053), for example, is commonly used (see. e,g., Stinchcomb L. et al. (1979) Nature, 282:39; Kingsman J. al. (1979), Gene, 7:141; S. Tschemper et al. (1980), Gene, 10:157). This plasmid already contains the trp gene which provides a selectable marker for a mutant strain of yeast lacking the ability to grow in tryptophan.

Suitable promoting sequences for use with yeast hosts include the promoters for 3-phosphoglycerate kinase (found on plasmid pAP12BD (ATCC 53231) and described in U.S. Pat. No. 4,935,350, issued Jun. 19, 1990, herein incorporated by reference) or other glycolytic enzymes such as enolase (found on plasmid pAC1 (ATCC 39532)), glyceraldehyde-3-phosphate dehydrogenase (derived from plasmid pHcGAPC1 (ATCC 57090, 57091)), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase, as well as the alcohol dehydrogenase and pyruvate decarboxylase genes of Zymomonas mobilis (U.S. Pat. No. 5,000,000 issued Mar. 19, 1991, herein incorporated by reference). Other yeast promoters, which are inducible promoters, having the additional advantage of their transcription being controllable by varying growth conditions, are the promoter regions for alcohol dehydrogenase 2, isocytochrome C, acid phosphatase, degradative enzymes associated with nitrogen metabolism, metallothionein (contained on plasmid vector pCL28XhoLHBPV (ATCC 39475) and described in U.S. Pat. No. 4,840,896, herein incorporated by reference), glyceraldehyde 3-phosphate dehydrogenase, and enzymes responsible for maltose and galactose (e.g. GAL1 found on plasmid pRY121 (ATCC 37658)) utilization. Yeast enhancers such as the UAS Ga1 from Saccharomyces cerevisiae (found in conjuction with the CYC1 promoter on plasmid YEpsec-hI1beta ATCC 67024), also are advantageously used with yeast promoters.

An aforementioned vector can be introduced into a host cell using any suitable method (e.g., transformation, electroporation, transfection using calcium chloride, rubidium chloride, calcium phosphate, DEAEdextran or other substances, microprojectile bombardment, lipofection, infection or transduction). Transformation relates to the introduction of DNA into an organism so that the DNA is replicable, either as an extrachromosomal element or by chromosomal integration. Methods of transforming bacterial and eukaryotic hosts are well known in the art. Numerous methods, such as nuclear injection, protoplast fusion or by calcium treatment are summerized in Sambrook J, Maniatis T (1989) *supra.* Transfection refers to the taking up of a vector by a host cell whether or not any coding sequences are in fact expressed. Successful transfection is generally recognized when any indication or the operation or this vector occurs within the host cell.

Alternatively, skilled artisans will recognize that portion (I) or other peptidic portions of the present inventive conjugate molecule can also be produced by a number of other methods. All amino acid sequences of the invention can be synthesized by chemical methods well known in the art. Including solid phase protein synthesis. Both methods are described in U.S. Pat. No. 4,617,149, the entirety of which is herein incorporated by reference. The principles of solid phase chemical synthesis of peptides are well known in the art and are described by, e.g., Dugas H. and Penney C. (1981), Bioorganic Chemistry, pages 54-92. For examples, proteins and peptides may be synthesized by solid-phase methodology utilizing an Applied Biosystems 430A peptide synthesizer (commercially available from Applied Biosystems, Foster City, Calif.) and synthesis cycles supplied by Applied Biosystems. Protected amino acids, such as t-butoxycarbonyl-protected amino acids, and other reagents are commercially available from many chemical supply houses. Sequential t-butoxycarbonyl chemistry using double couple protocols are applied to the starting p-methyl benzhydryl amine resins for the production of C-terminal carboxamides. For the production of C-terminal acids, the corresponding pyridine-2-aldoxime methiodide resin is used. Asparagine, glutamine, and arginine are coupled using preformed hydroxy benzotriazole esters. The following side chain protection may be used:
Arg, Tosyl; Asp, cyclohexyl; Glu, cyclohexyl; Ser, Benzyl; Thr, Benzyl; Tyr, 4-bromo carbobenzoxy
Removal of the t-butoxycarbonyl moiety (deprotection) may be accomplished with trifluoroacetic acid (TFA) in methylene chloride. Following completion of the synthesis the proteins or peptides may be deprotected and cleaved from the resin with anhydrous hydrogen fluoride containing 10% meta-cresol. Cleavage of the side chain protecting group(s) and of the peptidic portion from the resin is carried out at zero degrees centigrade or below, preferably -20°C. for thirty minutes followed by thirty minutes at 0 °C. After removal of the hydrogen fluoride, the peptide/resin is washed with ether, and the peptide extracted with glacial acetic acid and then lyophilized. Purification is accomplished by size-exclusion chromatography on a Sephadex G-10 (Pharmacia) column in 10% acetic acid. Subsequently, if an L-amino acid peptide is used as a matrix for D-enantiomeric-retro-inverso peptidic portions, e.g. portion (I), they are synthesized as described above by solid phase synthesis methods.

It should be appreciated, that the invention refers to clinical applications and may also be advantageously applied in medical and biological research. In order to make the present invention amenable to clinical use a pharmaceutical composition is provided which comprises a conjugate molecule of the invention as therapeutic compound and optionally a pharmaceutically acceptable carrier, adjuvant and/or vehicle. This pharmaceutical composition is intended for the treatment of cancer diseases/neoplastic conditions/tumors. Preferably, these diseases include e.g., tumors of the lymphatic system, like Hodgkin lymphoma, non-Hodgkin lymphoma, histocytic lymphoma, cancers of the brain (e.g. glioblastomas), ovarian, genitourinary tract, colon, liver, colorectal tract, bone, respiratory tract, eye, pancreas, breast, prostate, , stomach, larynx and lung, including lung adenocarcinoma and small cell lung cancer and/or skin, e.g. melanoma or non-melanoma skin cancer, including basal cell and squamous cell carcinomas. Likewise, the present invention includes a method for treating a condition, e.g. one of the afore-mentioned conditions, comprising the steps of administering a therapeutically effective amount of a conjugate molecule according to the invention.

Another embodiment of the present invention also relates to therapeutic methods or to the use of the inventive conjugate molecules or pharmaceutical compositions containing inventive conjugate molecules for the treatment or for the preparation of medicaments for the treatment of cancer, e.g., tumors of the lymphatic system, like Hodgkin lymphoma, non-Hodgkin lymphoma, histocytic lymphoma, acute or chronic leukemea, cancers of the brain (e.g. glioblastomas), ovarian, genitourinary tract, colon, liver, colorectal tract, pancreas, breast, prostate, eye, bone, respiratory tract, stomach, larynx and lung, including lung adenocarcinoma and small cell lung cancer and/or skin, e.g. melanoma or non-melanoma skin cancer, including basal cell and squamous cell carcinomas. Alternatively, the inventive conjugate molecule may be used for the treatment or the preparation of a medicament for the treatment of viral, bacterial or protozoological infectious diseases, in particular retroviral infections, e.g. HIV infections, or HCV infections. Likewise, the present invention includes a method for treating a condition, e.g. one of the afore-mentioned conditions, comprising the steps of administering a therapeutically effective amount of a conjugate molecule according to the invention.

A "pharmaceutically acceptable carrier, adjuvant, or vehicle" according to the invention refers to a non-toxic carrier, adjuvant or vehicle that does not destroy the pharmacological activity of the inventive conjugate molecule as therapeutic compound with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethyl cellulose, polyacrylates, waxes, polyethylene-polyoxypropylene-block polymers, polyethylene glycol and wool fat.

The pharmaceutical composition of the present invention may be administered parentally or non-parentally (e.g. orally).

The term "parenteral" as used herein includes subcutaneous, intravenous, intramuscular, intra-articular, intra-synovial, intraperitoneally, intrasternal, intrathecal, intrahepatic, intralesional and intracranial injection or infusion techniques. If administered parentally, the pharmaceutical compositions are administered preferably subcutaneously or intravenously. Sterile injectable forms of the pharmaceutical compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

If administered orally, the pharmaceutical compositions of this invention may be administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavouring or colouring agents may also be added. Additionally, standard pharmaceutical methods can be employed to control the duration of action. These are well known in the art and include control release preparations and can include appropriate macromolecules, for example polymers, polyesters, polyaminoacids, polyvinylpyrrolidone, ethylenevinylacetate, methyl cellulose, caraboxymethyl cellulose or protamine sulfate. The concentration of macromolecules as well as a the methods of incorporation can be adjusted in order to control release. Additionally, the agent can be incorporated into particles of polymeric materials such as polyesters, polyaminoacids, hydrogels, poly (lactic acid) or ethylenevinylacetate copolymers. In addition to being incorporated, these agents can also be used to trap the compound in microcapsules.

Further administration forms are e.g. by inhalation spray, topically, rectally, nasally, buccally, vaginally or via an implanted reservoir, some of which are described in the following in more detail.

Accordingly, the pharmaceutical compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

The pharmaceutical compositions of this invention may also be administered topically, especially when the target of treatment includes areas or organs readily accessible by topical application, including diseases of the eye, the skin, or the lower intestinal tract. Suitable topical formulations are readily prepared for each of these areas or organs. Topical application for the lower intestinal tract can be effected in a rectal suppository formulation (see above) or in a suitable enema formulation. Topically-transdermal patches may also be used. For topical applications, the pharmaceutical compositions may be formulated in a suitable ointment containing the active component suspended or dissolved in one or more carriers. Carriers for topical administration of the conjugate molecules of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active conjugate molecules suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutical compositions may be formulated in an ointment such as petrolatum.

The pharmaceutical compositions of this invention may also be administered by nasal aerosol or inhalation. Such pharmaceutical compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Most preferably, the pharmaceutical compositions of this invention are formulated for oral administration.

The amount of the conjugate molecule(s) of the present invention that may be combined with carriers, adjuvants and vehicles to produce a pharmaceutical composition in a single dosage form will vary depending upon the host treated, the particular mode of administration. Preferably, the pharmaceutical compositions should be formulated so that a dosage of between 0.01-100 mg/kg body weight/day of the inhibitor can be administered to a patient receiving these compositions. Preferred dosages range from 0.1 - 5 mg/kg body weight/day, even further preferred dosages from 1 - 5 mg/kg body weight/day.

Useful pharmaceutical dosage forms for administration of the compounds of this invention can be illustrated as follows.

Capsules: Capsules are prepared by filling standard two-piece hard gelatin capsulates each with 100 milligram of powdered active ingredient, 175 milligrams of lactose, 24 milligrams of talc and 6 milligrams magnesium stearate. Soft Gelatin Capsules: A mixture of active ingredient in soybean oil is prepared and injected by means of a positive displacement pump into gelatin to form soft gelatin capsules containing 100 milligrams of the active ingredient. The capsules are then washed and dried. Tablets: Tablets are prepared by conventional procedures so that the dosage unit is 100 milligrams of active ingredient. 0.2 milligrams of colloidal silicon dioxide, 5 milligrams of magnesium stearate, 275 milligrams of microcrystalline cellulose, 11 milligrams of cornstarch and 98.8 milligrams of lactose. Appropriate coatings may be applied to increase palatability or to delay absorption. Injectable: A parenteral composition suitable for administration by injection is prepared by stirring 1.5% by weight of active ingredients in 10% by volume propylene glycol and water. The solution is made isotonic with sodium chloride and sterilized. Suspension: An aqueous suspension is prepared for oral administration so that each 5 millimeters contain 100 milligrams of finely divided active ingredient, 200 milligrams of sodium carboxymethyl cellulose, 5 milligrams of sodium benzoate, 1.0 grams of sorbitol solution U.S.P. and 0.025 millimeters of vanillin.

It has to be noted that a specific dosage and treatment regimen for any particular patient will depend upon a variety of factors, including the activity of the specific conjugate molecule employed, the age, body weight, general health, sex, diet, time of administration, rate of excretion, drug combination, and the judgment of the treating physician and the severity of the particular disease being treated. The amount of conjugate molecules of the present invention in the pharmaceutical composition will also depend upon the particular conjugate molecule in the composition.

The terms used herein shall be interpreted in the following way. The term "therapeutic" as used here, for example, in the terms "therapeutic compound" and "therapeutically effective amount" means to have at least some minimal physiological effect. For example, a "therapeutic compound" would have at least some minimal physiological effect upon being administered to a living body. An agent may have at least some minimal physiological effect upon administration to a living body if, for example, its presence results in a change in the physiology of a recipient animal. For example, a physiological effect upon administering a "therapeutic" anti-tumor compound may be the inhibition of tumor growth, or decrease in tumor size, or prevention reoccurrence of the tumor. Administration of a "therapeutically effective amount" means the amount administered is physiologically significant. An agent is physiologically significant if its presence results in a change in the physiology of a recipient animal. For example, in the treatment of cancer or neoplastic disease, a compound which inhibits the growth of a tumor or decreased the size of the tumor or prevents the reoccurrence of the tumor would be considered therapeutically effective. The term "anti-tumor drug" means any therapeutic agent having therapeutic effect against a tumor, neoplastic disease or cancer. The term "drug" means any agent having a therapeutic effect when administered to an animal. The dosage of the present administration for therapeutic treatment will be sufficient to generate a therapeutically effective amount of the administered agent. The term "condition" means any condition, state, disease, abnormality, imbalance, malady and the like in an animal which one seeks to effect by administrating a therapeutically effective amount of a therapeutic compound. A condition is meant to include cancer/neoplastic diseases/tumors, and related conditions. The term "treating", used for example in the term "treating a condition", means at least the administration of a therapeutically effective amount of a therapeutic compound to elicit a therapeutic effect. It does not necessarily imply "curing", but rather having at least some minimal physiological effect upon a condition upon administration to a living body having a condition. For example, treatment could encompass administering an agent and the presence of that agent resulting in a change in the physiology of a recipient animal.

Further embodiments of the present invention relate to a method for improving the cell permeability or intracellular retention time of a anti-cancer drug or a protease inhibitor moiety by covalently conjugating the drug moiety with at least one drug carrier moiety, thereby creating a therapeutic compound, whereby the therapeutic compound is a conjugate molecule according to the invention. By the method according to the invention the portion of drug molecules being lovated intracellularly may be enhanced considerably. Preferably, a method according to the invention uses a conjugate molecule, wherein the drug carrier moiety has a molecular weight in the range of about 1.000 daltons to about 50.000 daltons. The method according to the invention allows to ensure that the inventive conjugate molecule has a cell permeability or intracellular retention time that is greater than the cell permeability or intracellular retention time of the anti-cancer or protease inhibitor drug moiety without the carrier moiety.

The following figures and examples are thought to illustrate the invention and should not be constructed to limit the scope of the invention thereon. All references cited by the disclosure of the present application are hereby incorporated in their entirety by reference.

### Figures

**Figure 1** shows the D amino acid sequence of a preferred carrier sequence which corresponds (in retro-inverso order) to amino acids 48-57 (a) and 49-57 (b) of HIV Tat (D-Tat sequences). All amino acids are D-enantiomeric amino acids (also termed herein as DR-DR-DR-DQ-DR-DR-DK-DK-DR (D-Tat sequence (b)).

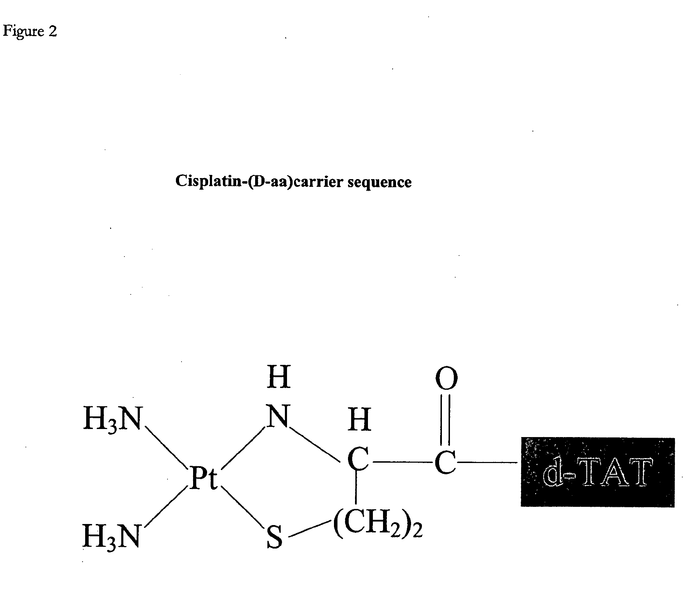

**Figure 2** shows an inventive conjugate molecule comprising cisplatin coupled covalently to a carrier sequence composed of D amino acids, e.g. a D-Tat sequence as shown in figure 1.

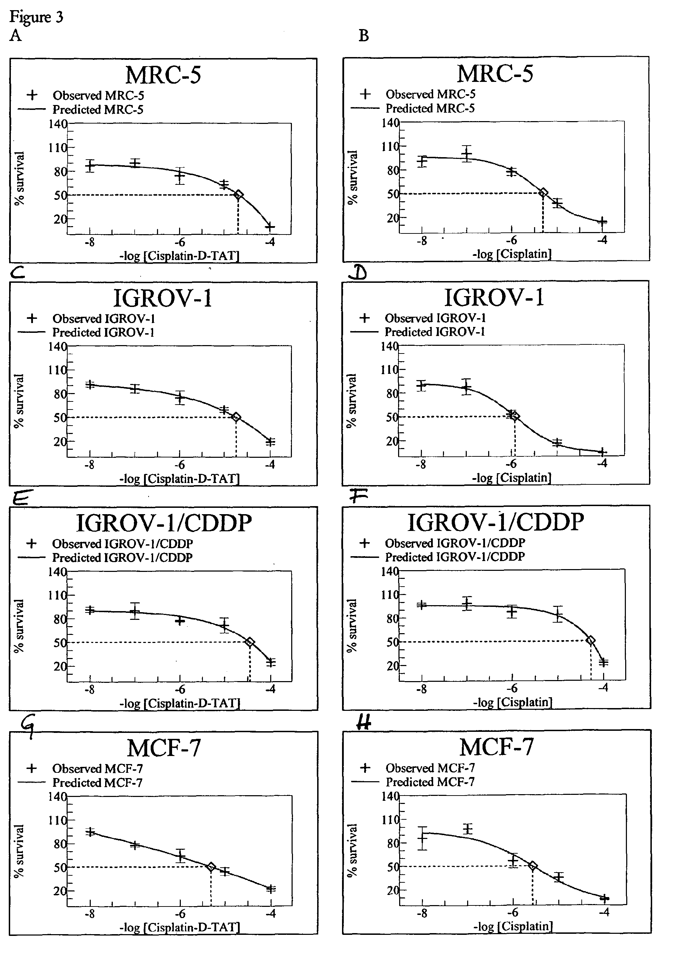

**Figure 3** shows the results of experiments summarizing the cell survival as a function of the concentration of either cisplatin or an inventive conjugate molecule consisting of cisplatin covalently coupled to a D amino acid sequence as shown in Figure 1 for four different cell lines (IGROV-1 (C/D), MRC-5 (A/B), IGROV-1/CDDP (E/F), MCF-7 (G/H). Pictures of the the right column (B, D, F, H) document the the comparative experiments with cisplatin, while the left column pictures reflect the effects of an inventive conjugate molecule. Negative logarithm is given on the x-axis, cell survival is indicated on the y-axis. IC50 values are indicated as well.

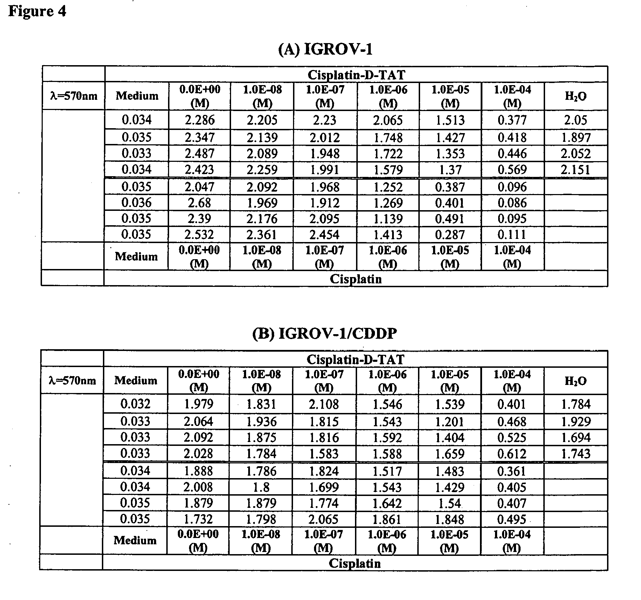

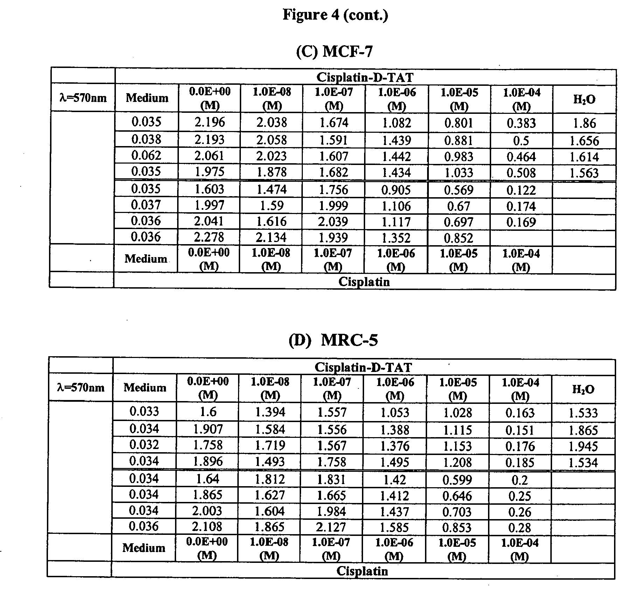

**Figure 4** is a summary of all experiments carried on for the cell line IGROV-1 (A), IGROV-1/CDDP (B), MCF-7 (C), and MRC-5 (D). The measured values for each single experiment with varying concentrations for cisplatin (lower part of each table) or cisplatin-D-Tat (upper part of each table) (concentration range 0 M (control) to 10⁻⁴ M) are given. Values measured for the medium and for water are indicated as well.

It is to be understood that these examples are in no way intended to limit the scope of the present invention but merely illustrate one example of a preferred embodiment presently known to the inventors. Additional, embodiments are within the scope of the present invention.

### Examples

### Example 1

### Synthesis of a conjugate molecule: D-Tat-cisplatin

### 1.1 Materials

Unless otherwise specified, all solvents and reagents were obtained from Fluka, Buchs, Switzerland, were of analytical or higher grade and were used without further purification. All amino acids and resin were purchased from NovaBiochem, Lucerne, Switzerland. Water was repurified using a Milli-Q system (Millipore, Inc.).

### 1.2 RP-HPLC

Analytical RP-HPLC was performed using a column 250 x 4 mm i.d. packed with Nucleosil 300-A 5 µm C₈ particles at a flow rate of 1.0 ml/min and effluent was monitored at 214nm. Semi-preparative peptide purification was carried out using a C₈ column (250 x 10 mm i.d. Nucleosil 300-A 5 µm particle size) at a flow rate of 4 ml/min monitoring at 214 nm. Solvents used in RP-HPLC were as follows: A, 0.1% TFA (1.0 g TFA in 1.0 litre dH₂O); B, 0.1% TFA in 90% acetonitrile (1.0 g TFA mixed with 100 ml H₂O and then brought to 1.0 litre with acetonitrile). Generally, the condition used in analytical work was a linear gradient 3% B/min to 100% B, and in Semi-preparative work a shallower linear gradient (usually 0.5% B/min) was used. Components were collected manually at the detector exit, partially evaporated at room temperature, frozen and then recovered by lyophilization.

### 1.3 Mass Spectrometry

Electrospray ionization mass spectrometry (ESI-MS) was performed in positive ion mode on a Platform II instrument (Micromass, Manchester, England). Samples were introduced at 10 µl/min in solvent acetonitrile/water/formic acid (49.9 : 49.9 : 0.2). External calibration was performed on the electrospray machines using a solution of horse heart apomyoglobin.

### 1.4 Peptide (D-TAT-Methionine) synthesis

The peptide sequence is DM-G-DR-DR-DR-DQ-DR-DR-DK-DK-DR, and the side-chain protection of Fmoc-protected amino acids were Arg(pbf), Gln(Trt) and Lys(Boc). The synthesis was performed manually on 0.4 mmol Fmoc-Amide-AM resin by using Fmoc chemistry. Thus, each amino acid from C-terminal D-Arg to N-terminal D-Met was sequencially attached to the resin with a cycle of Fmoc deprotection (20% piperidine in DMF) and amino acid coupling (TBTU/HOBt/DIEA activation). The peptide was cleaved from the resin with TFA (5h in the presence of 2.5% dH₂O, 2.5% EDT and 1% TIS), filtered under a reduced pressure, precipitated with cold ether, and lyophilized. The crude peptide was purified by Semi-preparative HPLC and characterized by ESI-MS.

### 1.5 Alkylation of peptide to Cisplatin

5.0 µmol of Cisplatin (1.5 mg in 3.0 ml Sodium Chloride buffer, pH 5.0) was dissolved in 2.0 ml of 10 mM Na₂HPO₄ buffer (pH 7.4), and pH value of the solution was 7.0. 5.0 µmol of D-TAT-Methionine peptide was prepared in 10 mM Na₂HPO₄ buffer (pH 7.4) and pH value of the solution was 6.0. Then the alkylation was started by mixing two solutions at room temperature in dark (pH value of the mixture was 7.0). After 0h, 1h, 3h and 24h, the product was analysed by analytic RP-HPLC, and characterized by ESI-MS. The expected peak solution was finally purified by Semi-preparative RP-HPLC and lyophilized.

### Example 2

### Comparative studies

### 2.1 Test conditions

Effects of a treatment with increasing concentrations of a conjugate molecule of the invention (Cisplatin-D-TAT) and an unconjugated anti-cancer drug (Cisplatin) on the survival of IGROV-1 (ovary), IGROV-1/CDDP (ovary, cisplatin-resistant cell line), MCF-7 (breast), and MRC-5 (human fibroblast) (three human tumor cell lines and one human fibroblast cell line) was determined. Cells of each cell line were plated out (200 µl final volume of RPMI 1640 supplemeted with 10 % FBS) in 96 well plates (5 different concentrations for each test substance, one control experiment). 5,000 to 40,000 cells per well (depending on the doubling time for each cell line) were incubated at 37°C for 24h before treatment with the test substances. Each experiment was carried 4x. Cell incubation after treatment was performed for 96 h at 37°C. The effects of Cisplatin-D-TAT and Cisplatin on the survival of these cell lines (in vitro cytotoxic activity) were measured by using an MTT assay. 20 µl of a 5 mg/ml solution 0,22 µl filtered tetrazolium reagent (MTT, Ref. M2003, Sigma) in Phosphate Buffered Saline (PBS, Ref BE17-517Q, Cambrex) were aded in each well. Culture plates were incubated for 4h at 37°C. The resulting supernatant was removed and formazan crystals were dissolved with 200 µl of DMSO per well. Absorbancy (OD) was determined in each well on a single wavelength spectrophotometer plate reader at 570 nm (Multiskan, Labsystem, Helsinki, Finland). Data were collected with Genesis software (Labsystem, Helsinki, Finland). IC50 (concentration of the drug inhibiting 50 % of the cell growth) for the test substances was calculated for each cell line after plotting. Control cells were treated with vehicle.

### 2.2 Results

The following results were obtained. Cell lines MRC-5, MCF-7, IGROV-1 and IGROV-1/CDDP tested with Cisplatin-D-TAT were sensitive to that substance with IC₅₀ ranging from 4.89 to 20.61 µM for MCF-7 and MRC-5 cell lines, respectively. IGROV-1/CDDP cell line (a cisplatin-resistant cell line) exhibited a resistance index of approximately two fold to the Cisplatin-D-TAT test substance when compared to the parental IGROV-1 cell line. All cell lines tested with Cisplatin (without the carrier portion D-Tat) were sensitive to that substance with IC₅₀ ranging from 1.20 to 52.32 µM for IGROV-1 and IGROV-1/CDDP cell lines, respectively. However, IGROV-1/CDDP cell line exhibited a resistance index of approximately 40 fold to the unconjugated cisplatin test substance when compared to the parental IGROV-1 cell line. It is clearly indicated that, while cisplatin-D-TAT and Cisplatin are active in approximately the same order of magnitude, cisplatin-resistant cell lines, like IGROV-1/CDDP, are more sensitive to the treatment with inventive conjugated cisplatin-D-Tat molecules. The results are shown in Table I and in Figure 4 below. The results are expressed as percentages of cell survival. Each value is the mean of four measurements.

**Table I:**

| **Cell lines** | **IC**_{**50**}**(µM)** | |
|---|---|---|
| | **Cisplatin-D-TAT** | **Cisplatin** |
| | | |
| **IGROV-1** | 18.39 | 1.20 |
| **IGROV-1/CDDP** | 35.30 | 52.32 |
| **MCF-7** | 4.89 | 2.68 |
| **MRC-5** | 20.61 | 4.88 |
| | | |

**Table I**: Resistance Index of IGROV-1/CDDP *versus* IGROV-1 cell line for **Cisplatin-D-TAT** and **Cisplatin** test substances.

| **Test substances** | **Resistance Index** |
|---|---|
| **Cisplatin-D-TAT** | 1.9 |
| **Cisplatin** | 43.6 |

## Claims

1. A conjugate molecule comprising at least one first portion (I) comprising a carrier sequence and at least one second portion (II) comprising a organic drug molecule selected from a group containing anti-cancer drug molecules and protease inhibitor molecules, said conjugate molecule comprising D-enantiomeric amino acids in its portion (I).

2. The conjugate molecule of claim 1, wherein the at least one first portion (I) and the at least one second portion (II) are linked by a covalent bond.

3. The conjugate molecule of any of claims 1 or 2, wherein portion (I) comprises a carrier sequence which directs the conjugate molecule to a defined cellular location.

4. The conjugate molecule of any of claims 1 to 3, wherein portion (I) comprises a carrier sequence which enhances cellular uptake of the conjugate molecule, in particular by enhancing cell permeability or by enhancing the intracellular retention time.

5. The conjugate molecule of any of claims 1 to 4, wherein portion (I) comprises as a carrier sequence a D peptide sequence according to one of general formulae (a) to (i) (a) NH2-Xm-COOH, (b) NH₂-XnArXn-COOH, (c) NH₂-XpAoXpAoXp-COOH, (d) NH₂-AoXpAoXpAo-COOH, (e) NH₂-XpAoXpAoXpAo-COOH, or (f) NH₂₋AoXpAoXpAoXpAoXpAo-COOH, or (g) NH₂- AoXpAoXpAoXpAoXpAoXpAo-COOH, (h) NH₂-AoXpAoXpAoXpAoXpAoXpAoXpAo-COOH, or (i) NH₂₋AoXpAoXpAoXpAoXpAoXpAoXpAoXpAo-COOH, whereby "X" is selected from D amino acids arginine or lysine, "m" is an integer between 3 and 40, preferably 3 and 20 and most preferably between 3 and 12, "A" relates to any non-basic D amino acid, "n" and "r" represents an integer from 1 to 20, preferably from 3 to 10 and more preferably from 3 to 6 amino acids, and "o" and "p" are integers from 0 to 14.

6. The conjugate molecule of any of claims 1 to 4, wherein portion (I) comprises a carrier sequence which is a D-TAT sequence (HIV Tat sequence in retro-inverso order composed of D amino acids) or a fragment thereof.

7. The conjugate molecule of claim 6, wherein portion (I) comprises the D amino acid sequence of DR-DR-DR-DQ-DR-DR-DK-DK-DR-DG or DR-DR-DR-DQ-DR-DR-DK-DK-DR).

8. The conjugate molecule of any of claims 1 to 7, wherein portion (II) comprises at least one protease inhibitor molecule or at least one anti-tumor drug molecule.

9. The conjugate molecule of any of claims 1 to 8, wherein portion (II) comprises at least one anti-tumor drug molecule selected from the group consisting of alkylating drugs, antimetabolica, cytostatics and drugs related to hormone treatment.

10. The conjugate molecule of any of claims 1 to 9, wherein portion (II) comprises at least one anti-tumor drug molecule selected from the group consisting of compounds of the taxol class or compounds and the class of platin derivatives.

11. The conjugate molecule of any of claims 1 to 10, wherein portion (II) comprises at least one anti-tumor drug molecule selected from the group consisting of cisplatin, satraplatin, oxaliplatin, carboplatin, and nedaplatin.

12. The conjugate molecule of any of claims 1 to 8, wherein portion (II) comprises at least one molecule of the class of protease inhibitor molecules selected from the group consisting of 640385, abacavir sulfate, AG1776, amprenavir (141W94 or VX-478), atazanavir ( BMS-232632), Cathepsin S protease inhibitor, D1927, D9120, efavirenz, emtricitabine, enfuvirtide (T-20), fosamprenavir (GW-433908 or VX-175), GS 9005, GW640385 (VX-385), HCV protease inhibitor, indinavir (MK-639), L-756, 423, levoprin-ZG, lopinavir (ABT-378), lopinavir/ritonavir (LPV ABT-378/r), MK-944A, mozenavir (DMP450), nelfinavir (AG-1343), nevirapine, P-1946, PL-100, prinomastat, ritonavir (ABT-538), RO033-4649, TMC114, saquinavir (Ro-31-8959), tenofovir disoproxil fumarate, tipranavir (PNU-140690), TLK 19781, TMC-114, Vertex 385, VX-950.

13. A pharmaceutical composition comprising a conjugate molecule of any of claims 1 to 12 and optionally a pharmaceutically acceptable carrier, adjuvant and/or vehicle.

14. Use of a conjugate molecule of any of claims 1 to 11 for the treatment and/or prophylaxis or for the preparation of a medicament for the treatment of cancer diseases, e.g., Hodgkin lymphoma, non-Hodgkin lymphoma, histocytic lymphoma, cancers of the brain (glioblastomas), ovarian, genitourinary tract, colon, liver, colorectal tract, pancreas, breast, prostate, lymphatic system, stomach, larynx and lung, including lung adenocarcinoma and small cell lung cancer, and/or skin, e.g. melanoma or non-melanoma skin cancer, including basal cell and squamous cell carcinomas.

15. Use of a conjugate molecule of claim 8 or 12 for the treatment and/or prophylaxis or for the preparation of a medicament for the treatment of viral, bacterial or protozoological infectious diseases, in particular HIV infections.

16. A method for improving the cell permeability or intracellular retention time of a anti-cancer drug or a protease inhibitor moiety by covalently conjugating the drug moiety with at least one drug carrier moiety, thereby creating a therapeutic compound, whereby the therapeutic compound is a conjugate molecule of any of claims 1 to 12.

17. The method of claim 16, wherein the drug carrier moiety comprises a molecular weight in the range of about 1.000 daltons to about 50.000 daltons.

18. The method of claim 16 or 17, wherein the cell permeability or intracellular retention time is greater than the the cell permeability or intracellular retention time of the anti-cancer or protease inhibitor drug moiety as such.

19. A method for treating a condition comprising the steps of: administering a therapeutically effective amount of a conjugate molecule of any of claims 1 to 12.
